# EUROPÄISCHE PATENTANMELDUNG

(11) **EP 3 511 874 A1**
(43) Veröffentlichungstag der Anmeldung: **17.07.2019**
(21) Anmeldenummer: 18151679.0
(22) Anmeldetag: 15.01.2018
(51) Int. Cl.: G06Q 10/00

(54) **KODIERUNG EINES CELLULOSEPRODUKTS**

(71) Anmelder: LENZING AKTIENGESELLSCHAFT, 4860 Lenzing (AT)
(72) Erfinder: Klaus-Nietrost, Christoph, 4840 Vöcklabruck (AT); Herchl, Richard, 4910 Ried im Innkreis (AT); Weilach, Christian, 4840 Vöcklabruck (AT)
(74) Vertreter: Dilg, Haeusler, Schindelmann Patentanwaltsgesellschaft mbH

(57) **Zusammenfassung**

Verfahren zum Identifizieren eines Cellulose aufweisenden Produkts (150), wobei bei dem Verfahren ein Datensatz (180) ermittelt wird, der für mehrere Bestandteile des Produkts (150) oder einer Vorform des Produkts (150) indikativ ist, und der mit dem Produkt (150) oder der Vorform korrelierte Datensatz (180) in einer Datenbank (152) hinterlegt wird.

## Beschreibung

Die Erfindung betrifft ein Verfahren zum Identifizieren eines Cellulose aufweisenden Produkts und eine Verwendung.

Als Viskosefasern werden Chemiefasern bzw. Regeneratfasern bezeichnet, die mittels eines Viskoseverfahren genannten Nassspinnverfahrens hergestellt werden. Der Ausgangsrohstoff des Viskoseverfahrens ist Cellulose, die auf der Basis von Holz bereitgestellt wird. Aus diesem Ausgangsrohstoff Holz wird die hochreine Cellulose in Form von Chemiezellstoff gewonnen. In aufeinanderfolgenden Prozessstufen wird der Zellstoff zuerst mit Natronlauge behandelt, wodurch sich Alkalicellulose bildet. Bei einer anschließenden Umsetzung dieser Alkalicellulose mit Schwefelkohlenstoff bildet sich Cellulose-Xanthogenat. Daraus wird durch weitere Zugabe von Natronlauge die Viskose-Spinnlösung erzeugt, die durch Löcher brausenartiger Spinndüsen in ein Spinnbad gepumpt wird. Dort entsteht durch Koagulation pro Spinndüsenloch ein Viskose-Filament. Die so hergestellten Viskose-Filamente werden im Anschluss zu Viskose-Stapelfasern geschnitten.

Lyocell bezeichnet eine Cellulose aufweisende Regeneratfasergattung, die nach einem Direkt-Lösemittelverfahren hergestellt wird. Die Cellulose wird für das Lyocell-Verfahren aus dem Rohstoff Holz extrahiert. Der so gewonnene Zellstoff kann anschließend in N-Methylmorpholin-N-oxid (NMMO), einem Lösungsmittel, durch Wasserentzug ohne chemische Modifizierung gelöst, filtriert und anschließend durch Spinndüsen gepresst werden. Die so geformten Filamente werden nach Passieren eines Luftspaltes in einem Bad mit wässriger NMMO-Lösung ausgefällt, und anschließend zu Stapelfasern geschnitten.

Gerade für Anwendungen, bei denen ein Produkt in Körperkontakt mit einem Benutzer gelangt (wie dies zum Beispiel bei Kleidungsstücken, Kosmetik oder medizintechnischen Produkten der Fall ist), ist es wichtig, eine zuverlässige Qualität von Cellulose aufweisenden Produkten sicherzustellen.

Es ist eine Aufgabe der vorliegenden Erfindung, eine zuverlässige Qualität von Cellulose aufweisenden Produkten sicherzustellen.

Diese Aufgabe wird durch die Gegenstände gemäß den unabhängigen Patentansprüchen gelöst. Bevorzugte Ausgestaltungen ergeben sich aus den abhängigen Patentansprüchen.

Gemäß einem Ausführungsbeispiel der vorliegenden Erfindung ist ein Verfahren zum Identifizieren eines Cellulose aufweisenden Produkts (wobei das Produkt insbesondere einen, weiter insbesondere regenerierten cellulosischen, Formkörper aufweisen kann) geschaffen, wobei bei dem Verfahren ein Datensatz ermittelt wird, der für mehrere Bestandteile des Produkts oder einer Vorform des Produkts indikativ ist, und der mit dem Produkt oder der Vorform korrelierte Datensatz in einer Datenbank hinterlegt wird.

Gemäß einem anderen Ausführungsbeispiel wird eine gemessene und in einer Datenbank hinterlegte Information hinsichtlich mindestens eines intrinsischen nichtcellulosischen Bestandteils (insbesondere einer Information hinsichtlich einer Kombination betreffend mehrere intrinsische nichtcellulosische Bestandteile) eines aus wiederverwerteten Alttextilien hergestellten Celluloseprodukts oder eines Formkörpers, aus dem das Celluloseprodukt hergestellt wurde, als Code zum Identifizieren einer Herkunft des Celluloseprodukts oder des Formkörpers verwendet.

Im Rahmen dieser Anmeldung kann unter dem Begriff "Cellulose" insbesondere eine organische Verbindung verstanden werden, die Bestandteil pflanzlicher Zellwände ist oder synthetisch hergestellt werden kann. Cellulose ist ein Polysaccharid (d.h. ein Vielfachzucker). Cellulose ist unverzweigt und weist typischerweise mehrere hundert bis zehntausende β-D-Glucose-Moleküle (β-1,4-glycosidische Bindung) bzw. Cellobiose-Einheiten auf. Aus Cellulosemolekülen werden von Pflanzen kontrolliert Cellulosefasern aufgebaut. Mit einem technischen Prozess können Cellulosemoleküle unter Bildung von Regeneratfasern zusammengelagert werden, zum Beispiel als reißfeste Fasern.

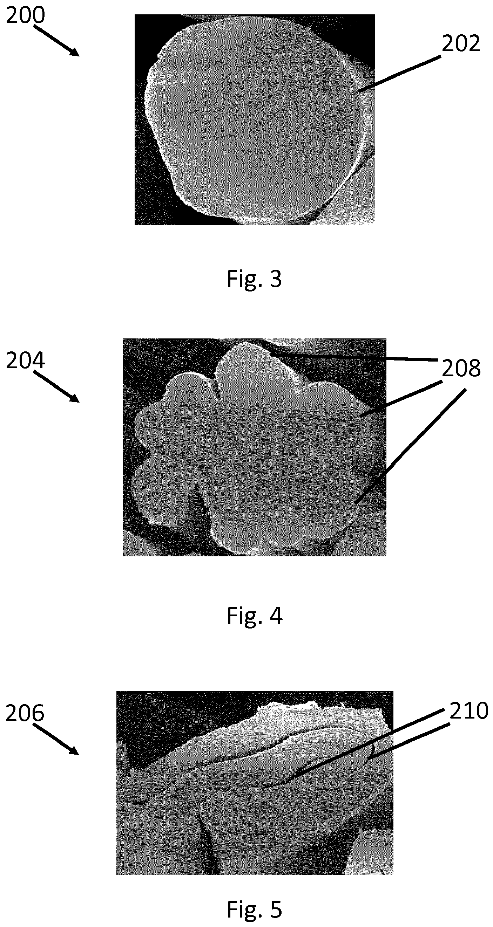

Im Rahmen dieser Anmeldung können unter dem Begriff "Bestandteile des Produkts" insbesondere unterschiedliche Materialien bzw. Komponenten oder Konstituenten des Cellulose aufweisenden Produkts verstanden werden, die messtechnisch unterscheidbar sind. Die Bestandteile des Produkts können einerseits von einem Ausgangsstoff zum Herstellen des Produkts abhängen und können andererseits von einem Verfahren zum Herstellen des Produkts abhängen. Die Kombination der Bestandteile und deren Mengen eines Produkts können daher indikativ sein für ein ganz bestimmtes Produkt oder eine ganz bestimmte Charge des Produkts oder einer Vorform desselben, das auf der Basis bestimmter Ausgangsstoffe mit einem bestimmten Verfahren hergestellt worden ist. Ein solcher Bestandteil kann zum Beispiel ein Hauptbestandteil des Produkts oder der Vorform sein (zum Beispiel Cellulose, die in unterschiedlichen Fasergeometrien auftreten kann - vergleiche Figur 3 bis Figur 5 - und die aufgrund unterschiedlicher Produktzusammensetzungen der verwendeten Cellulosequellen auch spektroskopisch unterscheidbar sein kann, zum Beispiel mittels Kristallinitätsbestimmung via NMR). Ein solcher Bestandteil kann aber auch ein Fremdstoff sein, der als Ergebnis der Verwendung bestimmter Ausgangsstoffe bzw. Cellulosequellen auch im Endprodukt enthalten ist (beispielsweise Polyester bzw. Elastan im Falle der Wiederverwertung von Altkleidern). Ein Bestandteil kann auch ein Spurenelement sein, das auf eine Cellulosequelle bzw. einen Ausgangsstoff zurückgeht (zum Beispiel Titandioxid oder ein Seltenerdmetall). Darüber hinaus ist es auch möglich, dass ein Bestandteil des Produkts analysiert wird, der im Ausgangsstoff bzw. der Cellulosequelle noch nicht enthalten war, sondern erst während oder nach Herstellung der Cellulosefasern oder eines anderen cellulosischen Formkörpers dem Produkt hinzugefügt wurde, beispielsweise ein Farbstoffmarker.

Im Rahmen dieser Anmeldung können unter dem Begriff "Datensatz" insbesondere (zum Beispiel elektronisch speicherbare) Daten verstanden werden, deren Dateninhalt für mehrere Bestandteile deren Vorhandensein bzw. Nichtvorhandensein in einem Produkt oder seiner Vorform oder sogar deren Menge in einem Produkt oder seiner Vorform angibt. Zum Beispiel kann der Datensatz eine Abfolge von logischen Werten "1" und logischen Werten "0" aufweisen, mithin einen Binärcode bzw. eine Bitfolge, der bzw. die bezogen auf ein bestimmtes Produkt oder die Vorform die Erfüllung oder Nichterfüllung eines jeweiligen Kriteriums angibt, das im Zusammenhang mit einem bestimmten Bestandteil steht. Zum Beispiel kann 1 Bit eines solchen Datensatzes angeben, ob ein Produkt eine Konzentration eines Metalloxids oberhalb (entsprechend einem logischen Wert "1") oder unterhalb (entsprechend einem logischen Wert "0") eines vorgegebenen Schwellwerts hat. Auf diese Weise kann mit einer kompakten Datenstruktur ein Fingerabdruck eines bestimmten Produkts oder seiner Vorform auf Basis seiner Bestandteile dargestellt werden.

Im Rahmen dieser Anmeldung kann unter dem Begriff "Datenbank" insbesondere eine Gesamtheit von Datensätzen (die zum Beispiel auf einem Datenspeicher, weiter insbesondere einem elektronischen Massenspeicher (zum Beispiel einer Festplatte) gespeichert sein können), verstanden werden. Eine solche Datenbank kann lokal bei einer Apparatur zum Herstellen von Cellulose aufweisenden Produkten bzw. Formkörpern oder auch an entfernter Stelle angeordnet sein, zum Beispiel Cloud-basiert sein.

Im Rahmen dieser Anmeldung kann unter dem Begriff "Vorform des Produkts" insbesondere ein Zwischenprodukt (insbesondere auch ein Halbzeug) verstanden werden, das während des Herstellens des Produkts (insbesondere eines Endprodukts, das zur Verwendung durch einen Verbraucher vorgesehen ist) erhalten wird und nach einer Weiterverarbeitung in das Produkt überführt werden kann. Vorform und Produkt können zumindest teilweise dieselbe Materialzusammensetzung haben und/oder zumindest teilweise aus denselben Bestandteilen gebildet sein. Zum Beispiel kann das Produkt eine Jeans sein und eine zugehörige Vorform eine Cellulose-Faser oder ein anderer Formkörper (insbesondere ein regenerierter cellulosischer Formkörper), aus dem die Jeans hergestellt ist.

Gemäß einem exemplarischen Ausführungsbeispiel der Erfindung ist ein Codiersystem für Celluloseprodukte geschaffen, bei dem nach dem Herstellen eines solchen Produkts oder einer Vorform davon messtechnisch Informationen zu vorzugsweise mehreren Bestandteilen dieses speziellen Produkts oder der Vorform davon erfasst werden und als Datensatz abgespeichert werden. Diese Datensatz kann diesem speziellen Produkt oder der Vorform davon zugeordnet werden. Diese Prozedur kann für viele Produkte und/oder Vorformen wiederholt werden. Der Datensatz für ein bestimmtes Produkt oder seine Vorform hängt maßgeblich von den Ausgangsstoffen (zum Beispiel Holz, Reste aus der Kleiderherstellung, Altkleider, natürliche Baumwolle, etc.) zum Herstellen des cellulosischen Produkts oder der Vorform sowie vom verwendeten Herstellungsverfahren (zum Beispiel Lyocell-Verfahren oder Viskoseverfahren) ab. Wenn zum Beispiel als Ausgangsstoffe ganz oder teilweise zu recycelnde inhomogene Mischtextile zum Einsatz kommen, deren Materialzusammensetzung für unterschiedliche Chargen stark schwankt, so kann für jedes Produkt bzw. jede Vorform ein individueller Fingerabdruck der Ausgangsstoffe und des Herstellungsverfahrens in Form des Datensatzes abgespeichert werden. Ein Textil kann hier insbesondere ein verarbeitetes Faserprodukt sein. Ein Mischtextil kann dementsprechend als inhomogenes Faserprodukt bezeichnet werden. Soll später, zum Beispiel aus Gründen der Qualitätssicherung, die Originalität eines bestimmten Testprodukts überprüft werden, können dieselben Parameter abermals messtechnisch erfasst werden und eine beste Übereinstimmung (zum Beispiel im Sinne eines Best-Match-Verfahrens) zwischen den erfassten Parameterwerten des Testprodukts und einem der abgespeicherten Datensätze gesucht werden. In dem einem bestimmten Produkt zugeordneten Datensatz ist damit die Produktidentität codiert. Indem gemäß einem exemplarischen Ausführungsbeispiel eine Mehrzahl von Bestandteilen in den zugehörigen Datensatz eingeht, kann die Wahrscheinlichkeit für einen falschen Treffer (d.h. ein Produkt wird irrtümlich einem falschen Datensatz zugeordnet) stark reduziert werden.

Besonders vorteilhaft ist es, eine Kombination aus mehreren intrinsischen nichtcellulosischen Bestandteilen eines aus wiederverwerteten Alttextilien hergestellten Celluloseprodukts als Fingerabdruck eines bestimmten Produkts oder einer Produkt-Vorform als zugehörigen Datensatz in einer Datenbank abzuspeichern und bedarfsweise mit einem Testprodukt abzugleichen. Unter einem intrinsischen Bestandteil kann in diesem Zusammenhang insbesondere ein Bestandteil des Produkts oder der Vorform verstanden werden, der als Ergebnis eines hierfür verwendeten Ausgangsstoffs (bevorzugt Alttextilien) und eines verwendeten Herstellungsverfahrens ohne zusätzliche Maßnahme zwangsläufig in dem finalen Produkt oder auch in einer Vorform desselben enthalten ist. Dadurch ist der Datensatz praktisch ohne Zusatzaufwand generierbar, da der als Marker verwendete Bestandteil dem Produkt nicht zwingend separat beigefügt werden muss, und dennoch spezifisch indikativ für ein ganz spezielles Produkt sein kann (da bei Alttextilien von inhomogenen Mischtextilien ausgegangen werden kann). Indem als analysierte Bestandteile gerade nicht oder zumindest nicht nur die Cellulose selbst verwendet wird, sondern zumindest auch aus den Alttextilien stammende nichtcellulosische Fremdstoffe, kann ein ganz besonders spezifischer bzw. individueller Datensatz für ein bestimmtes Produkt abgespeichert und mit Testprodukten abgeglichen werden. Auf diese Weise ist es möglich, eine zuverlässige Qualität von Cellulose aufweisenden Produkten sicherzustellen, indem die Herkunft der Produkte bzw. deren Hervorgehen aus einem Wiederverwertungsverfahren verifiziert und Fälschungen oder Plagiate zuverlässig identifiziert werden können. Auf diese Weise ist auch für Zelluloseprodukte (zum Beispiel Kleidung) eine Nachverfolgbarkeit ermöglicht, ohne dass für Celluloseprodukte nicht oder nur schwer einsetzbare und aufwendige Maßnahmen (wie das Anbringen von Barcode oder RFID-Tags) ergriffen werden müssten

Im Weiteren werden zusätzliche Ausführungsbeispiele des Verfahrens und der Verwendung beschrieben.

Gemäß einem Ausführungsbeispiel kann der ermittelte Datensatz dafür indikativ sein, ob das Produkt oder die Vorform zumindest teilweise auf Basis einer wiederverwerteten Cellulosequelle als Ausgangsstoff zum Herstellen des Produkts oder der Vorform hergestellt wurde. Im Rahmen dieser Anmeldung kann unter dem Begriff "Cellulosequelle" insbesondere ein Medium (insbesondere ein Festkörpermedium) verstanden werden, das als Basis zum Herstellen eines Cellulose aufweisenden Formkörpers während eines entsprechenden Herstellungsverfahrens das hierfür verwendete Cellulosematerial bereitstellt. Ein Beispiel ist Holz bzw. Holzzellstoff. Andere Beispiele für eine wiederverwertete Cellulosequelle sind wiederzuverwertende Textilabfälle, wie Post-Consumer Altkleider oder Pre-Consumer Reste aus der Textilherstellung. Bei der Verwendung von wiederzuverwertendem Material als Ausgangsstoff bzw. Cellulosequelle zum Herstellen des Cellulose aufweisenden Produkts oder seiner Vorform finden sich in dem hergestellten Produkt oder der Vorform des Produkts zwangsläufig Substanzen, die in der Cellulosequelle enthalten waren. Wenn zum Beispiel die Cellulosequelle aus Altkleidern gebildet ist, können sich synthetische Kunststoffe wie Polyester oder Elastan an und/oder in den hergestellten Cellulosefasern befinden. Solche synthetischen Kunststoffe finden in Kleidungsstücken in Form von Nähten, Knöpfen, bzw. Beimischungen zu Cellulosefasern Anwendung. Da beim Wiederverwerten von Altkleidern oder dergleichen häufig inhomogene Mischtextilien Anwendung finden (die jeweils zur Verfügung stehenden Altkleider haben keine feste Zusammensetzung, vielmehr wird in der Regel ein gerade zur Verfügung stehender Mix weiterverarbeitet), ist in dem genannten Anwendungsfall die Zusammensetzung eines hergestellten Cellulose aufweisenden Produkts von Charge zu Charge signifikant unterschiedlich. Eine nach Herstellung eines solchen Produkts oder einer Vorform messtechnisch erfasste Zusammensetzung mehrerer Bestandteile des Produkts oder der Vorform kann daher als Markerinformation auch für die genannte Charge und für das Zurückgehen eines Produkts oder einer Vorform (zum Beispiel einen Formkörper) auf einen bestimmten Betrieb bzw. eine bestimmte Herstellungsapparatur verwendet werden. Die Verwendung von Cellulosequellen im Wege der Wiederverwertung von Mischtextilien bietet also besondere und ohne zusätzlichen Aufwand verwertbare Vorteile im Zusammenhang mit der Schaffung eines Codiersystems für Cellulose aufweisende Produkte.

Gemäß einem bevorzugten Ausführungsbeispiel kann das Produkt oder die Vorform zumindest teilweise auf Basis von Altkleidern als Cellulosequelle als Ausgangsstoff zum Herstellen des Produkts oder der Vorform hergestellt werden. Zum Beispiel können bei dem Ausgangsstoff mindestens 3 Gewichtsprozent, insbesondere mindestens 10 Gewichtsprozent, weiter insbesondere mindestens 50 Gewichtsprozent, Altkleider bezogen auf das Gesamtgewicht des Ausgangsstoffs verwendet werden. Besonders vorteilhaft können zur Herstellung des Produkts wiederverwertete Alttextilien von einem Benutzer getragene Kleidungsstücke aufweisen oder daraus bestehen. Ein solches Ausführungsbeispiel ist besonders deshalb bevorzugt, da Altkleider häufig eine besonders inhomogene Zusammensetzung mit erheblichen Anteilen von Fremdstoffen aufweisen, die als intrinsische nichtcellulosische Bestandteile des herzustellenden Produkts als Marker verwendet werden können. Im Rahmen dieser Anmeldung können unter dem Begriff "Altkleider" insbesondere Cellulose aufweisende Kleidungsstücke verstanden werden, die bei Rückgewinnung zumindest eines Teils der Cellulose bereits von einem Verbraucher benutzt (insbesondere getragen) worden sind. Bei Altkleidern kann es sich also um einen Cellulose aufweisenden Ausgangsstoff handeln, der erhebliche Mengen von Fremdstoffen aufweisen kann (aber nicht muss) und zum Wiedergewinnen von Cellulose verwendet werden kann, nachdem ein Verbraucher die Altkleider als Kleidung oder in anderer Weise verwendet hat. Altkleider können insbesondere aus einer Mischung aus Cellulose und einem oder mehreren Fremdstoffen gebildet sein, insbesondere aufweisend (insbesondere bei Kleidungsstücken häufig verwendeten) synthetischen Kunststoff (wie zum Beispiel Polyester und/oder Elastan) und/oder separate und nicht Cellulose aufweisende Fremdkörper (wie zum Beispiel Knöpfe, Textildruck oder Nähte). Unter Polyester werden insbesondere Polymere mit Esterfunktionen (R-[-CO-O-]-R) in ihrer Hauptkette verstanden. Zu Polyestern gehören Polycarbonate und Polyethylenterephthalat. Unter Elastan wird insbesondere eine dehnbare Chemiefaser mit hoher Elastizität verstanden. Ein Elastan zugrundeliegendes Blockcopolymer kann einen Massenanteil von mindestens 85 % Polyurethan enthalten.

Gemäß einem Ausführungsbeispiel kann das Produkt oder die Vorform alternativ oder ergänzend zumindest teilweise auf Basis von Resten aus einer Kleidungsherstellung als Cellulosequelle als Ausgangsstoff zum Herstellen des Produkts oder der Vorform hergestellt werden. Im Rahmen dieser Anmeldung kann unter dem Begriff "Reste aus einer Kleidungsherstellung" insbesondere Ausschuss und/oder Verschnitt eines Cellulose aufweisenden oder daraus bestehenden Textils oder Garnes verstanden werden, wobei diese Reste während eines Verfahrens zum Herstellen von Kleidung anfallen. Beim Herstellen von Kleidung wird zum Beispiel ein Cellulose aufweisendes Textil als Ausgangsstoff hergestellt, aus dem dann flächige Teile (zum Beispiel in Form einer T-Shirt-Hälfte) ausgeschnitten werden. Übrig bleiben Reste, die gemäß einem exemplarischen Ausführungsbeispiel wieder einem Verfahren zum Herstellen eines Cellulose aufweisenden Formkörpers zugeführt werden können. Bei Resten aus einer Kleidungsherstellung kann es sich also um einen Cellulose aufweisenden oder daraus bestehenden Ausgangsstoff handeln, der zum Wiedergewinnen von Cellulose verwendet werden kann, bevor ein Verbraucher die Reste als Kleidung oder in anderer Weise verwendet hat. Reste aus einer Kleidungsherstellung können insbesondere aus im Wesentlichen reiner Cellulose gebildet sein, insbesondere ohne separate und nicht Cellulose aufweisende Fremdkörper (wie zum Beispiel Knöpfe, Textildruck oder Nähte).

Gemäß einem Ausführungsbeispiel kann das Produkt oder die Vorform zumindest teilweise auf Basis von einer weiteren Cellulosequelle hergestellt werden, insbesondere auf Basis von zumindest einem Material aus einer Gruppe, die besteht aus Holzzellstoff, Hadernzellstoff (insbesondere Zellstoff aus Stoffresten wie Zuschnittsresten, etc.), Baumwolle (d.h. Cellulose aus einer Baumwollpflanze), mittels eines Lyocell-Verfahrens hergestellter Cellulose und/oder mittels eines Viskoseverfahrens hergestellter Cellulose. Es ist also auch möglich, nur einen Teil des Ausgangsstoffs in Form von Altkleidern bzw. Resten aus der Textilherstellung bereitzustellen. Der Ausgangsstoff kann auch ein inhomogenes Mischgewebe sein, was seine Eignung als Codiermarker weiter verbessert. Der Rest des Ausgangsstoffs, der nicht in Form von Altkleidern bzw. Resten aus der Textilherstellung zugeführt wird, kann flexibel und je nach Verfügbarkeit durch eine oder mehrere andere Cellulosequellen aufgefüllt werden. Dadurch wird der großtechnische Einsatz des Herstellungsverfahrens nicht durch etwaige vorübergehende Engpässe an Altkleidern bzw. Resten aus der Textilherstellung beeinträchtigt. Vielmehr ist es möglich, etwaige Fehlmengen an Cellulose aus wiederzuverwertenden Textilien durch andere Cellulosequellen zu kompensieren bzw. auszugleichen. Bevorzugt kann aber das Bilden des Ausgangsstoffs nur aus Altkleidern sein, da deren Eigenschaften als inhomogenes Mischtextil für ein intrinsisches Codiersystem gemäß einem exemplarischen Ausführungsbeispiel besonders vorteilhaft sind.

Gemäß einem Ausführungsbeispiel kann das Produkt oder die Vorform auf Basis eines Ausgangsstoffs hergestellt werden, der als inhomogene (und optional über einen Zeitraum eines oder mehrerer Herstellungsverfahren hinweg zeitlich veränderliche) Mischung unterschiedlicher Bestandteile hergestellt wird. Gerade diese Inhomogenität, die durch unvorhersehbare Eigenschaften von eintreffendem wiederzuverwertenden Material als Ausgangsstoff zustande kommt, kann gemäß einem exemplarischen Ausführungsbeispiel vorteilhaft dazu genutzt werden, dass auch unterschiedliche Produkte bzw. Vorformen infolge der inhomogenen Ausgangsstoffe zumindest auf der Ebene von Fremdstoffen stark voneinander abweichen, sodass eben diese Fremdstoffe mit Vorteil zur Kodierung bzw. Identifizierung von Produkten und deren Vorformen eingesetzt werden können.

Gemäß einem Ausführungsbeispiel kann das Produkt oder die Vorform mittels des Lyocell-Verfahrens oder mittels des Viskoseverfahrens hergestellt werden. Sowohl beim Lyocell-Verfahren (vergleiche Figur 3) als auch beim Viskoseverfahren (vergleiche Figur 4) können Cellulosefasern charakteristische geometrische Eigenschaften aufgeprägt werden. Daher kann die Faserform von Cellulose in dem hergestellten Produkt als Teil des Datensatzes bzw. einer zugehörigen Bitfolge einen Teil des Codes bzw. Fingerabdrucks eines Produkts aus einer bestimmten Charge und aus einer bestimmten Herstellungsapparatur sein.

Im Rahmen dieser Anmeldung kann unter dem Begriff "Lyocell-Verfahren" insbesondere ein Verfahren zum Herstellen von Cellulose nach einem Direkt-Lösemittelverfahren verstanden werden. Die Cellulose kann für das Lyocell-Verfahren aus einem Ausgangsstoff gewonnen werden, der diese Cellulose enthält. Der Ausgangsstoff kann bei dem Lyocell-Verfahren in einem geeigneten Lösungsmittel (insbesondere aufweisend tertiäre Aminoxide wie zum Beispiel N-Methylmorpholin-N-oxid (NMMO) und/oder ionische Flüssigkeiten, d.h. niedrig schmelzende Salze, die aus Kationen und Anionen aufgebaut sind) gelöst werden. Das Lösen kann insbesondere durch Wasserentzug und/oder ohne chemische Modifizierung erfolgen. Die erhaltene Lösung, die auch als Dope oder Spinnlösung bezeichnet werden kann, kann bei dem Lyocell-Verfahren anschließend durch eine oder mehrere Spinndüsen gepresst werden. Dadurch gebildete Filamente können während und/oder nach ihres freien oder gesteuerten Fallens durch einen Luftspalt in einem wasserhaltigen Bad (insbesondere in einem Bad mit wässriger NMMO-Lösung) und/oder in dem Luftspalt befindliche Luftfeuchtigkeit ausgefällt werden.

Im Rahmen dieser Anmeldung kann unter dem Begriff "Viskoseverfahren" insbesondere ein Verfahren zum Herstellen von Cellulose nach einem Nassspinnverfahren verstanden werden. Die Cellulose kann für das Viskoseverfahren aus einem Ausgangsstoff (insbesondere Holz oder ein Holzzellstoff) gewonnen werden, der diese Cellulose enthält. In aufeinanderfolgenden Prozessstufen kann bei dem Viskoseverfahren der Ausgangsstoff zuerst mit einer Base (zum Beispiel mit Natronlauge) behandelt werden, wodurch sich Alkalicellulose bildet. Bei einer anschließenden Umsetzung dieser Alkalicellulose mit Schwefelkohlenstoff bildet sich Cellulose-Xanthogenat. Daraus kann durch weitere Zugabe einer Base (insbesondere von Natronlauge) eine Viskose-Spinnlösung erzeugt werden, die durch eine oder mehrere Spinndüsen gepresst werden kann. In einem Spinnbad entstehen durch Koagulation Viskose-Filamente.

Gemäß einem Ausführungsbeispiel kann bei dem Verfahren ein Testprodukt zum Ermitteln von Information analysiert werden, die für mehrere Bestandteile des Testprodukts indikativ ist, und die Information mit dem Datensatz in der Datenbank abgeglichen werden. Ein solches Testprodukt kann zum Beispiel ein Produkt sein, das während eines Vertriebs- bzw. Verwendungsprozesses auf seine Echtheit bzw. seinen Ursprung hin überprüft werden soll. Dies kann beispielsweise zur Klärung der Frage der Originalität eines Produkts oder seiner Vorform aus einer bestimmten Quelle, zur Zuordnung eines Produkts oder seiner Vorform zu einem bestimmten Herstellungsprozess (insbesondere einer bestimmten Charge), zur Nachverfolgung eines Produkts oder seiner Vorform über seine Lebensdauer hinweg, zur Klärung, ob ein Produkt oder seine Vorform aus einer wiederverwerteten Cellulosequelle stammt, etc., wünschenswert sein.

Gemäß einem Ausführungsbeispiel kann bei dem Verfahren mindestens ein anderer Datensatz ermittelt werden, der für mehrere Bestandteile zumindest eines anderen Produkts oder einer Vorform des zumindest einen anderen Produkts indikativ ist, und kann der mit dem zumindest einen anderen Produkt oder seiner Vorform korrelierte Datensatz in der Datenbank hinterlegt werden. Mit anderen Worten können für viele oder sogar alle hergestellten Produkte bzw. Vorformen Datensätze erfasst und in der Datenbank im Zusammenhang mit einem jeweiligen Produkt hinterlegt werden. Zur späteren Identifizierung eines Testprodukts als zu einem der Datensätze zugehörig kann dann auf viele Vergleichsdaten zurückgegriffen werden und daher die Zuverlässigkeit einer Zuordnung verbessert werden. Anders ausgedrückt wird durch einen solchen komplexen Satz vieler Datensätze die Informationsgrundlage für die Zuordnung eines Testprodukts verbreitert.

Gemäß einem Ausführungsbeispiel kann die Information mit den Datensätzen abgeglichen werden und kann das Testprodukt als einem der Datensätze zugehörig identifiziert werden, wenn eine Diskrepanz zwischen der Information einerseits und einem der Datensätze andererseits einen vorgegebenen Schwellwert, insbesondere eine Anzahl von nicht erfüllten Bitkriterien (vorzugsweise kein einziges unterschiedliches Bitkriterium), unterschreitet oder nicht überschreitet. Ein alternatives Entscheidungskriterium, ob einer der Datensätze mit einem Testprodukt übereinstimmt, besteht darin, dass eine Identität dann angenommen werden kann, wenn die Information und dieser Datensatz eine bestmögliche Übereinstimmung zeigen (insbesondere eine bessere Übereinstimmung als alle anderen Datensätze). Zum Beispiel kann eine bestmögliche Übereinstimmung zu einer höchsten Anzahl von übereinstimmenden Bitkriterien (vorzugsweise eine Übereinstimmung hinsichtlich aller Bitkriterien) korrespondieren. Wenn ein Testprodukt identifiziert bzw. dessen Ursprung nachverfolgt werden soll, kann das Testprodukts derselben Messung unterzogen werden, mit der für die Produkte bzw. die Vorformen die jeweiligen Datensätze gewonnen wurden. Zum Beispiel kann eine solche Aufklärung der mehreren Bestandteile und ihrer Eigenschaften (zum Beispiel ihrer Menge oder ihrem Vorhandensein oder Nichtvorhandensein) in einem Testprodukt durch eine spektroskopische Analyse (beispielsweise mittels Massenspektrometrie) erfolgen. Ein dadurch erhaltener Testdatensatz für das Testprodukt kann mit den in der Datenbank gespeicherten Datensätzen abgeglichen bzw. verglichen werden. Hierfür können mehrere Kriterien angewendet werden, die im Zusammenhang mit den analysierten Bestandteilen stehen können. Zum Beispiel kann das Kriterium angewandt werden, ob eine bestimmte Seltene Erde in dem Testprodukt ermittelt werden konnte oder ob eine bestimmte Seltene Erde mit einer bestimmten Mindestkonzentration oder innerhalb eines vorgegebenen Konzentrationsbereichs im Testprodukt gefunden werden konnte. Als Ergebnis der Anwendung dieser Kriterien auf das Testprodukt und durch einen entsprechenden Vergleich mit der Anwendung dieser Kriterien auf die Produkte bzw. die Vorformen gemäß den Datensätzen kann dann zum Beispiel mit Vorteil für jedes Kriterium ein zugehöriger Bitwert definiert werden (beispielsweise ein logischer Wert "1", falls das jeweilige Kriterium erfüllt ist, oder "0", falls das jeweilige Kriterium nicht erfüllt ist). Dadurch kann mit einer sehr geringen Anforderung an speichertechnische und prozessortechnische Ressourcen und folglich sehr schnell ein Abgleich der Bitwerte bzw. einer daraus aufgebauten Bitsequenz durchgeführt werden. Als Ergebnis dieser Rechenoperationen kann ein Datensatz aus der Datenbank extrahiert werden, der eine beste Übereinstimmung mit einem Datensatz des Testprodukts ergab. Eine Zuordnung eines Testprodukts zu einem bestimmten Produkt (oder zu einer Vorform des Produkts), für das seinerzeit ein zugehöriger Datensatz abgespeichert wurde, ist daher in fehlerrobuster Weise möglich.

Gemäß einem Ausführungsbeispiel kann der Datensatz für eine Kombination von in dem Produkt vorhandenen Bestandteilen, insbesondere für eine Kombination von in dem Produkt in einer jeweiligen Menge enthaltenen Bestandteilen, indikativ sein. Je höher die Zahl der Bestandteile, die in den Datensatz Eingang finden, desto aussagekräftiger ist das Ergebnis einer Zuordnung zwischen einem Produkt entsprechend einem Datensatz und einem zu untersuchenden Testprodukt. Zum Beispiel kann die Anzahl von Bestandteilen, zu denen in der Datenbank ein entsprechendes Kriterium oder ein entsprechender Wert verankert ist, mindestens drei, insbesondere mindestens vier, weiter insbesondere mindestens sechs, bevorzugt mindestens acht, sein. Je höher die Anzahl von Bestandteilen, desto geringer die Gefahr, dass ein Testprodukt einem falschen Datensatz zugeordnet wird.

Gemäß einem Ausführungsbeispiel können sich die mehreren Bestandteile, insbesondere ausschließlich, auf nicht-cellulosische Bestandteile des Produkts oder der Vorform beziehen. Unter "nicht-cellulosischen Bestandteilen" können hierbei Bestandteile bzw. Bestandteile des Produkts oder der Vorform verstanden werden, die von Cellulose (insbesondere Lyocell-Cellulose, Viskose und Baumwollcellulose) unterschiedlich sind. Bei diesen nicht-cellulosischen Komponenten kann sich also insbesondere um Fremdstoffe handeln, die in dem Ausgangsstoff enthalten waren (zum Beispiel Seltene Erden, Metalloxide, synthetische Kunststoffe, Farbstoffe, etc.) oder noch nicht in dem Ausgangsstoff enthalten waren und erst zu einem späteren Zeitpunkt während des Herstellens des Produkts als externer oder extrinsischer Marker beigefügt wurden (zum Beispiel eine Beschichtung aus Luminophoren).Als (insbesondere extrinsische) Marker können auch formselektierte Partikel eingesetzt werden, zum Beispiel würfel-, kugel-, und/oder tetraederförmige Nanopartikel. Da solche Fremdstoffe uncharakteristisch und unspezifisch für Celluloseprodukte sind, ohne die Qualität der Produkte bzw. der Vorform zu beeinträchtigen, eignen sie sich besonders gut als Marker oder Codierstoffe zum eindeutigen Identifizieren eines bestimmten Produkts oder zumindest einer bestimmten Produktcharge.

Gemäß einem Ausführungsbeispiel können sich die mehreren Bestandteile, insbesondere ausschließlich, auf intrinsische Bestandteile des Produkts oder der Vorform beziehen, die bereits nach einem Koagulieren von Cellulose aufweisenden Filamenten aus einer Spinnlösung in und/oder an und/oder zwischen den Filamenten enthalten waren. Insbesondere können die intrinsischen Bestandteile bereits nach einer Extrusion einer Spinnlösung zu cellulosischen Formkörpern in und/oder an den Formkörpern enthalten gewesen sein. Unter "intrinsischen Bestandteilen" können hierbei insbesondere Bestandteile des Produkts oder der Vorform verstanden werden, die bereits in dem Ausgangsstoff bzw. der Cellulosequelle zum Herstellen des Produkts oder der Produkt-Vorform enthalten waren. Aufgrund der in der Praxis starken Inhomogenität hinsichtlich der Zusammensetzung von Recycling-Ausgangsstoffen eignen sich intrinsische Bestandteile hervorragend zur Verwendung als Codiermarker, und dies ohne jeglichen Zusatzaufwand.

Gemäß einem Ausführungsbeispiel können die intrinsischen Bestandteile und/oder die nicht-cellulosischen Bestandteile zumindest einen aus einer Gruppe aufweisen, die besteht aus einem Spurenelement (d.h. ein Konstituent, der nur zu einem äußerst geringen Anteil enthalten ist), insbesondere einem Metall, weiter insbesondere einem Seltenerdmetall, einem Metalloxid, insbesondere Titandioxid und/oder Zinkoxid, einem Farbstoff, einem Leuchtstoff, und einem synthetischen Kunststoff, insbesondere Elastan und/oder Polyester. Andere intrinsische Bestandteile sind abhängig von der Zusammensetzung eines Ausgangsstoffs möglich und können besonders bei der Verwendung von Altkleidern als inhomogenes Mischtextil in unterschiedlichen Produktchargen hinsichtlich Art und Menge mit Vorteil stark schwanken.

Gemäß einem Ausführungsbeispiel kann sich zumindest einer der mehreren Bestandteile auf mindestens einen cellulosischen Bestandteil des Produkts oder der Vorform beziehen. Unter einem "cellulosischen Bestandteil" kann hierbei insbesondere ein Konstituent bzw. ein Bestandteil des Produkts oder der Vorform verstanden werden, der dessen Cellulose (insbesondere nach dem Lyocell- oder Viskoseverfahren regenerierte Cellulose, Baumwollcellulose und/oder Holzzellstoff) betrifft. Cellulose kann sich in unterschiedlichen Produkten, unterschiedlichen Vorformen oder in unterschiedlichen Produktbestandteilen hinsichtlich der geometrischen Erscheinungsform (vergleiche Figur 3 bis Figur 5) unterscheiden. Auch die chemischen Eigenschaften von Cellulose bzw. von deren Elementen (insbesondere Kohlenstoff) kann sich aufgrund der in unterschiedlichen Produkten unterschiedlichen chemischen Umgebung unterscheiden. Dies kann zum Beispiel in Form unterschiedlicher Verschiebungswerte in einem NMR Spektrum unterschieden werden. Der Datenbankabgleich kann also auch Informationen über einen Fasertyp eines Produkts oder einer Vorform liefern.

Gemäß einem Ausführungsbeispiel können die intrinsischen Bestandteile und/oder können die nicht-cellulosischen Bestandteile ganz oder teilweise aus Alttextilien stammen. Aufgrund der häufig hohen Inhomogenität und wechselnden Zusammensetzung von Alttextilien als wiederzuverwertender Ausgangsstoff zum Herstellen des zellulosehaltigen Produkts ist dessen Fremdstoffzusammensetzung ein besonders individueller Marker, der ein bestimmtes Produkt codiert und zuverlässig von anderen Produkten oder zumindest Produktchargen unterscheidet.

Gemäß einem Ausführungsbeispiel kann das Verfahren während oder nach einem Herstellen des Produkts oder seiner Vorform (insbesondere nach einem Koagulieren von Cellulose aufweisenden Filamenten aus einer Spinnlösung) ein Versetzen des Produkts oder einer Vorform des Produkts mit mindestens einem extrinsischen Bestandteil aufweisen, für den der in der Datenbank hinterlegte Datensatz ebenfalls indikativ ist. Ein externer oder extrinsischer Konstituent, der noch nicht in der Cellulosequelle als Ausgangsstoff zum Herstellen von Produkt bzw. Vorform enthalten war, kann dem Produkt also zur weiteren Verfeinerung der Codiergenauigkeit speziell zugesetzt werden. Das Versetzen der fertig hergestellten Cellulose des Produkts oder der Vorform mit einem extrinsischen Bestandteil (wie zum Beispiel einen Luminophor oder dergleichen) kann alternativ oder vorzugsweise ergänzend zum Messen mindestens eines intrinsischen Bestandteils des Produkts oder seiner Vorform die Unterscheidbarkeit des Produkts von anderen Produkten weiter verbessern. Ein solcher extrinsischer Bestandteil (oder mehrere) kann unterschiedlichen Produkten oder Vorformen hinsichtlich Typ und/oder Menge in gleicher oder vorzugsweise in unterschiedlichen Mengen beigefügt werden (zum Beispiel als im Formkörper inkorporiertes Pigment), um den Datensatz weiter zu verfeinern bzw. zu diversifizieren.

Gemäß einem Ausführungsbeispiel kann der Datensatz einer Produktionscharge zugeordnet werden. Gemäß einer solchen Ausführungsform ist es ausreichend, für alle Produkte einer Produktcharge (zum Beispiel alle an einem Tag an derselben Herstellungsapparatur hergestellten Produkte) gemeinsam einen Datensatz zu hinterlegen. Dies hält die zu verwaltende Datenmenge gering und ermöglicht ebenfalls eine sehr spezifische Charakterisierung eines Produkts hinsichtlich seines Ursprungs.

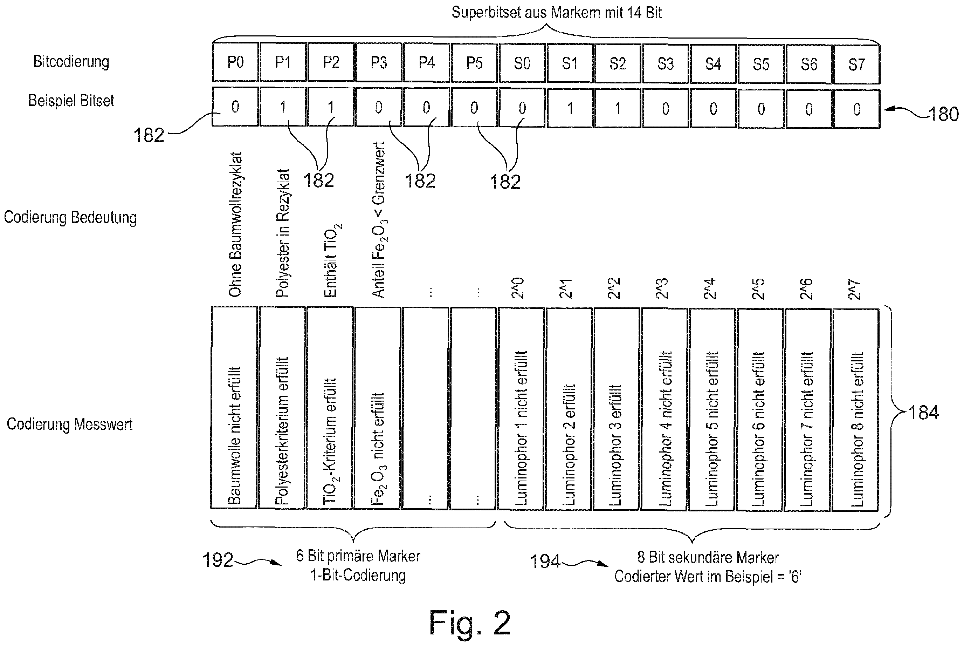

Gemäß einem Ausführungsbeispiel kann bei dem Verfahren als Datensatz eine für eine Zusammensetzung des Produkts oder seiner Vorform aus den mindestens zwei Bestandteilen indikative Bitfolge in der Datenbank hinterlegt werden (vergleiche Figur 2). Je länger die Bitfolge, umso höher ist die Zuverlässigkeit der Zuordnung eines Datensatzes zu einem Testprodukt.

Gemäß einem Ausführungsbeispiel kann das Produkt oder dessen Vorform ein Formkörper sein oder auf Basis eines Formkörpers hergestellt werden. Im Rahmen dieser Anmeldung kann unter dem Begriff "Formkörper" insbesondere ein zwei- oder dreidimensionaler geometrischer Körper verstanden werden, der ein Resultat eines Verfahrens zum Herstellen bzw. Wiedergewinnen von Cellulose ist. Insbesondere kann unter einem Formkörper ein zwei- oder dreidimensionales Objekt verstanden werden, das Cellulose aufweist oder daraus besteht und aus gelöstem Zellstoff hergestellt wird. Insbesondere kann der Formkörper ein regenerierter cellulosischer Formkörper sein. Formkörper können insbesondere Lyocell-Formkörper, Viskose-Formkörper oder Modal-Formkörper sein. Typische Formkörper sind Filamente, Fasern, Schwämme und/oder Filme. Grundsätzlich eignen sich alle Arten von Celluloseformkörpern für Ausführungsbeispiele der Erfindung. Dabei sind als Fasern sowohl Endlosfilamente als auch geschnittene Stapelfasern mit herkömmlichen Abmessungen (zum Beispiel 38 mm Länge) und Kurzfasern zu verstehen. Zur Herstellung von Fasern kommen dabei sowohl Verfahren mit Abzugseinrichtungen nach einer oder mehreren Extrusionsdüsen als auch andere Verfahren, wie insbesondere Melt-Blowing-Verfahren, in Frage. Alternativ zu Fasern kann als Formkörper auch eine Cellulose aufweisende Folie hergestellt werden, d.h. ein flächiger und im Wesentlichen homogener Film mit oder aus Cellulose. Folien können insbesondere dadurch hergestellt werden, dass durch Einstellung der Prozessparameter eines Lyocell-Verfahrens ein Koagulieren zumindest teilweise erst nach Auftreffen der Filamente auf einer Aufnahmefläche ausgelöst wird. Unter Folien können flächige Celluloseformkörper verstanden werden, wobei die Dicke dieser Folien einstellbar ist (zum Beispiel durch Auswahl einer Anzahl seriell angeordneter Düsenbalken). Andere Ausführungsformen eines Formkörpers sind ein Gewebe und ein Vlies aus Cellulosefilamenten bzw. aus Cellulosefasern, insbesondere ein Spinnvlies aus integral miteinander verschmolzenen ("merging") im Wesentlichen kontinuierlichen Cellulosefilamenten ("melt blown"). Hierbei kann unter einem Gewebe insbesondere ein textiles Flächengebilde aus mindestens zwei (vorzugsweise rechtwinklig oder nahezu rechtwinklig) verkreuzten Fadensystemen (oder Fasersystemen) verstanden werden, wobei Fäden (oder Fasern) in Längsrichtung als Kettfäden und Fäden (oder Fasern) in Querrichtung als Schussfäden bezeichnet werden können. Ein Vlies oder Nonwoven kann als ungeordnetes (insbesondere in Wirrlage vorliegendes) Gebilde aus Filamenten oder Fasern oder geschnittenen Garnen begrenzter Länge bezeichnet werden, die zu einer Faserschicht oder einem Faserflor zusammengefügt und (insbesondere reibschlüssig) miteinander verbunden sind. Ein Formkörper kann auch in der Gestalt einer Kugel geschaffen werden. Als Formkörper können auch Cellulose aufweisende Partikel, wie insbesondere Beads (d.h. ein Granulat bzw. Kügelchen) oder Flakes, bereitgestellt werden, die in dieser Form weiterverarbeitet werden können. Mögliche Celluloseformkörper sind also auch partikuläre Strukturen wie Granulat, sphärische Pulver oder Fibride. Eine Formgebung eines Formkörpers erfolgt bevorzugt durch die Extrusion einer cellulosehaltigen Spinnlösung durch eine Extrusionsdüse, da auf diese Weise große Mengen der Celluloseformkörper mit sehr einheitlicher Form hergestellt werden können. Ein weiterer möglicher Celluloseformkörper ist ein Schwamm oder allgemeiner ein poröser Formkörper. Die genannten Formkörper können gemäß exemplarischen Ausführungsbeispielen zum Beispiel zur Herstellung von Garnen, Textilien, Gelen oder Verbundwerkstoffen verwendet werden.

Die erfindungsgemäß hergestellten Formkörper können zum Beispiel als Verpackungsmaterial, Fasermaterial, Textilverbundstoffe, Faserverbundstoffe, Faservliese, Nadelfilze, Polsterwatte, Gewebe, Gestricke, als Heimtextilien, wie Bettwäsche, als Kleidungsstücke, als Füllstoff, Beflockungsstoff, Krankenhaustextilien, wie Unterlagen, Windel oder Matratzen, als Stoff für Wärmedecken, Schuheinlagen, sowie Wundverbände verwendet werden. Ausführungsbeispiele der Erfindung können sowohl in verschiedensten technischen Bereichen als auch in der Medizin und in Kosmetik und Wellness anwendbar sein. In der Medizin können zum Beispiel Materialien zur Wundbehandlung und Wundheilung aus einem Träger, der die mechanischen Eigenschaften bestimmt, und einem biokompatiblem Beschichtungsmaterial aufgebaut sein, das besonders verträglich mit der Haut und mit der Oberfläche der Wunde ist. Zahlreiche andere Anwendungen sind möglich.

Im Folgenden werden exemplarische Ausführungsbeispiele der vorliegenden Erfindung mit Verweis auf die folgenden Figuren detailliert beschrieben.
Figur 1 zeigt eine Apparatur zum Herstellen eines Cellulose aufweisenden Formkörpers mittels eines Lyocell-Verfahrens und zum nachfolgenden Identifizieren eines entsprechend hergestellten Cellulose aufweisenden Produkts während dessen Lebensdauer gemäß einem exemplarischen Ausführungsbeispiel der Erfindung.
Figur 2 zeigt eine Bitfolge eines Datensatzes, der ein Produkt mit einer mittels eines Lyocell-Verfahrens hergestellten Cellulosefaser identifiziert.
Figur 3 zeigt eine mittels eines Lyocell-Verfahrens hergestellte Cellulosefaser.
Figur 4 zeigt eine mittels eines Viskoseverfahrens hergestellte Cellulosefaser.
Figur 5 zeigt eine natürliche Cellulosefaser aus einer Baumwollpflanze.

Gleiche oder ähnliche Komponenten in unterschiedlichen Figuren sind mit gleichen Bezugsziffern versehen.

Bevor bezugnehmend auf die Figuren exemplarische Ausführungsbeispiele beschrieben werden, sollen einige grundlegende Überlegungen zusammengefasst werden, basierend auf denen exemplarische Ausführungsbeispiele der Erfindung abgeleitet worden sind.

Gemäß einem exemplarischen Ausführungsbeispiel der Erfindung wird ein Codiersystem für Celluloseprodukte, insbesondere hergestellt mittels eines Lyocell-Verfahrens, geschaffen. Auf diese Weise kann eine fälschungssichere Faser (als Formkörper oder Produkt-Vorform) bzw. einen fälschungssicheres, auf dieser Faser basierendes Produkt bereitgestellt werden, da ein vermeintliches Plagiat anhand des Codiersystems einfach und zuverlässig auf Echtheit überprüft werden kann. Genauer gesagt wird gemäß einem exemplarischen Ausführungsbeispiel ein System geschaffen, mit dessen Hilfe insbesondere cellulosische Lyocell-Fasern entsprechend markiert werden können, so dass beispielsweise ein Endkunde mit einem Prüfgerät (zum Beispiel ein spezielles Messgerät) in der Lage ist, die Echtheit einer angeblichen Lyocell-Faser (insbesondere hergestellt durch Recycling von Altkleidern) zu überprüfen. Beispielsweise kann eine Bitsequenz generiert und abgespeichert werden, welche charakteristische Daten einer Faser bzw. eines zugehörigen Produkts codiert. Mit Vorteil können die Bits durch unabhängige, physikalisch messbare Größen repräsentiert werden. Die Bits können dabei beispielsweise durch das Vorhandensein von bestimmten, üblicherweise nicht in textilen Flächengebilden vorkommenden Stoffen gesetzt werden (wie zum Beispiel Seltenerdmetalle, etc.). Es können aber alternativ oder ergänzend auch Reststoffe, welche aus Recyclingmaterialien nicht rückstandsfrei entfernt werden, zur Markierung eingesetzt werden. So kann zum Beispiel eine bestimmte Bitfolge an Recycling-Residuen als Bitfolge zur Kennzeichnung von Lyocell-Fasern mit Recycling-Anteil eingesetzt werden.

Gemäß einem Ausführungsbeispiel kann zum Beispiel eine Markierung von Lyocell-Fasern über einen Code, beispielsweise in Form einer Bitfolge, abgebildet werden, so dass eine Nachverfolgbarkeit der Fasern bis ins fertige Textilprodukt möglich ist. Dadurch kann eine zuverlässige Fälschungssicherung, insbesondere von Lyocell-Fasern, erreicht werden.

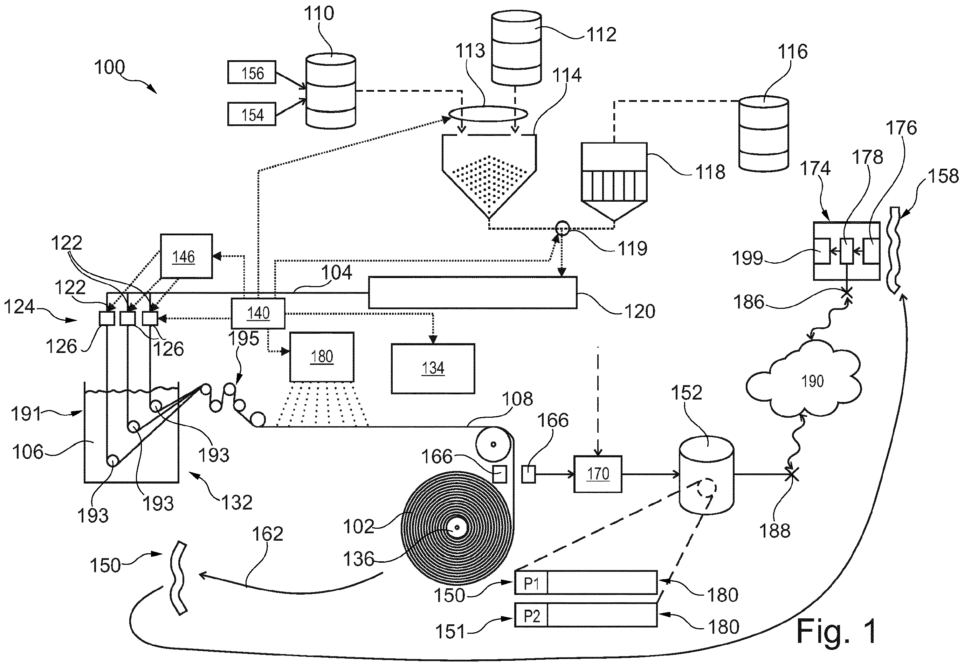

**Figur 1** zeigt eine Apparatur 100 zum Herstellen eines Cellulose aufweisenden Formkörpers 102 mittels eines Lyocell-Verfahrens und zum nachfolgenden Identifizieren eines entsprechend hergestellten Cellulose aufweisenden Produkts 150 während dessen Lebensdauer gemäß einem exemplarischen Ausführungsbeispiel der Erfindung.

Figur 1 zeigt also eine Apparatur 100 gemäß einem exemplarischen Ausführungsbeispiel der Erfindung zum Herstellen eines Cellulose aufweisenden Formkörpers 102, der zum Beispiel in Form eines Vlieses (Nonwoven), als Faser, Folie, Kugel, Textilgewebe, Schwamm oder in Form von Beads oder Flakes hergestellt werden kann. Gemäß Figur 1 wird der Formkörper 102 direkt aus einer Spinnlösung 104 hergestellt. Letztere wird mittels eines Koagulations-Fluids 106 (insbesondere aus Luftfeuchtigkeit) und/oder eines Koagulations-Bads 191 (beispielsweise ein Wasserbad, das optional tertiäre Aminoxide wie zum Beispiel N-Methylmorpholin-N-oxid (NMMO) aufweist) in Cellulosefasern 108 als Formkörper 102 umgewandelt. Mittels der Apparatur 100 kann ein Lyocell-Verfahren ausgeführt werden. Auf diese Weise können als Formkörper 102 zum Beispiel im Wesentlichen endlose Filamente oder Fasern 108 oder Mischungen im Wesentlichen endloser Filamente und Fasern 108 diskreter Länge hergestellt werden. Eine Mehrzahl von Düsen, die jeweils eine oder mehrere Öffnungen 126 (die auch als Spinnlöcher bezeichnet werden können) haben, sind bereitgestellt, um Lyocell-Spinnlösung 104 auszustoßen.

Wie Figur 1 entnommen werden kann, kann einem Speichertank 114 über eine Dosiereinrichtung 113 ein Cellulose basierter Ausgangsstoff 110 zugeführt werden.

Gemäß einem Ausführungsbeispiel kann ein Wassereintrag in den Cellulose basierten Ausgangsstoff 110 durch ein unten näher beschriebenes Lösungsmittel 116 (insbesondere NMMO) erfolgen. Auch kann der Cellulose basierte Ausgangsstoff 110 selbst bereits eine gewisse Restfeuchte enthalten (trockener Zellstoff zum Beispiel hat häufig eine Restfeuchte von 5 Gewichtsprozent bis 8 Gewichtsprozent). Insbesondere kann gemäß dem beschriebenen Ausführungsbeispiel der Ausgangsstoff 110 ohne Vorbefeuchtung direkt in eine Mischung aus Wasser und Lösungsmittel 116 gegeben werden. Ein in Figur 1 gezeigter optionaler Wasserbehälter 112 kann dann weggelassen werden.

Gemäß einem alternativen Ausführungsbeispiel kann der Cellulose aufweisende Ausgangsstoff 110 zusätzlich angefeuchtet werden, um dadurch feuchte Cellulose bereitzustellen. Zu diesem Zweck kann Wasser aus einem optionalen Wasserbehälter 112 dem Speichertank 114 über die Dosiereinrichtung 113 zugeführt werden. Daher kann die Dosiereinrichtung 113, gesteuert mittels einer Steuereinrichtung 140, dem Speichertank 114 einstellbare relative Mengen von Wasser und Ausgangsstoff 110 zuführen.

Ein geeignetes Lösungsmittel 116, bevorzugt tertiäre Aminoxide wie zum Beispiel N-Methylmorpholin-N-oxid (NMMO), beziehungsweise eine wässrige Mischung des Lösungsmittels 116, zum Beispiel eine 76%ige Lösung von NMMO in Wasser, ist in einem Lösungsmittelbehälter enthalten. Die Konzentration des Lösungsmittels 116 kann in einer Konzentriereinrichtung 118 entweder durch Zugabe von reinem Lösemittel oder Wasser eingestellt werden. Das Lösungsmittel 116 kann dann mit dem Ausgangsstoff 110 mit definierbaren relativen Mengen in einer Mischeinheit 119 gemischt werden. Auch die Mischeinheit 119 kann mittels der Steuereinheit 140 gesteuert werden. Dadurch wird der Cellulose aufweisende Ausgangsstoff 110 in dem konzentrierten Lösungsmittel 116 in einer Löseeinrichtung 120 mit einstellbaren relativen Mengen gelöst, wodurch die Lyocell-Spinnlösung 104 erhalten wird.. Die relativen Konzentrationsbereiche (auch als Spinnfenster bezeichnet) der Komponenten Ausgangsstoff 110, Wasser und Lösungsmittel 116 in der Spinnlösung 104 zur Herstellung von cellulosischen Regeneratformkörpern nach dem Lyocell-Verfahren können, wie einem Fachmann bekannt, geeignet eingestellt werden.

Die Lyocell-Spinnlösung 104 wird einer Fasererzeugungseinrichtung 124 zugeführt (die mit einer Anzahl von Spinnbalken oder Jets 122 ausgebildet sein kann).

Wenn die Lyocell-Spinnlösung 104 durch die Öffnungen 126 der Jets 122 geführt wird, wird sie in eine Mehrzahl von parallelen Fäden aus Lyocell-Spinnlösung 104 aufgeteilt. Die beschriebene Prozessführung transformiert die Lyocell-Spinnlösung 104 in zunehmend lange und dünne Fäden, deren Eigenschaften durch entsprechende Einstellung der Prozessbedingungen, gesteuert durch die Steuereinheit 140, eingestellt werden können. Optional kann ein Gasfluss die Lyocell-Spinnlösung 104 auf ihrem Weg von den Öffnungen 126 zu einer Faseraufnahmeeinheit 132 beschleunigen.

Nachdem die Lyocell-Spinnlösung 104 sich durch die Jets 122 und weiter abwärts bewegt hat, interagieren die langen und dünnen Fäden der Lyocell-Spinlösung 104 mit dem Koagulations-Fluid 106.

Bei der Wechselwirkung mit dem Koagulations-Fluid 106 (zum Beispiel Wasser) wird die Lösungsmittelkonzentration der Lyocell-Spinnlösung 104 reduziert, sodass die Cellulose des Ausgangsstoffs 110 zumindest teilweise als lange und dünne Cellulosefasern 108 (die immer noch Reste von Lösungsmittel und Wasser enthalten können) koaguliert bzw. ausfällt.

Während oder nach der initialen Bildung der individuellen Cellulosefasern 108 aus der extrudierten Lyocell-Spinnlösung 104 werden die Cellulosefasern 108 an der Faseraufnahmeeinheit 132 aufgenommen. Die Cellulosefasern 108 können in das in Figur 1 dargestellte Koagulations-Bad 191 (zum Beispiel ein Wasserbad, optional aufweisend ein Lösungsmittel wie NMMO) eintauchen und können bei Wechselwirkung mit der Flüssigkeit des Koagulations-Bads 191 ihr Ausfällen abschließen. Abhängig von der Prozesseinstellung der Koagulation kann die Cellulose Cellulosefasern 108 bilden (wie gezeigt, wobei die Cellulosefasern 108 einstoffig bzw. integral miteinander verschmolzen ("merging") sein können oder als getrennte Cellulosefasern 108 vorliegen können) oder kann sich an der Faseraufnahmeeinheit 132 eine Folie bzw. ein Film aus Cellulose bilden (nicht in Figur 1 dargestellt).

Die Cellulosefasern 108 werden also aus den Spinndüsen der Jets 122 extrudiert und durch das Spinnbad bzw. Koagulations-Bad 191 (enthaltend zum Beispiel Wasser und NMMO in niedriger Konzentration zur Ausfällung/Koagulation) geführt, dabei werden die Cellulosefasern 108 um eine jeweilige Umlenkrolle 193 im Koagulations-Bad 191 geführt und außerhalb des Koagulations-Bads 191 einer Abzugsgalette 195 zugeführt. Die Abzugsgalette 195 sorgt für Weitertransport und Nachverstreckung der Cellulosefasern 108, um einen gewünschten Titer zu erreichen. Nach der Abzugsgalette 195 wird das Faserbündel aus den Cellulosefasern 108 in einer Wascheinheit 180 gewaschen, gegebenenfalls aviviert und schließlich geschnitten (nicht gezeigt).

Obwohl dies in Figur 1 nicht dargestellt ist, kann Lösungsmittel 116 der Lyocell-Spinnlösung 104, das aus den Cellulosefasern 108 beim Koagulieren und bei einem nachfolgenden Waschen in der Wascheinheit 180 entfernt worden ist, zumindest teilweise zurückgewonnen bzw. recycelt werden und in einem nachfolgenden Zyklus wieder in den Speichertank 114 übergeführt werden.

Während des Transports entlang der Faseraufnahmeeinheit 132 kann der Formkörper 102 (hier in Form der Cellulosefasern 108) mittels der Wascheinheit 180 gewaschen werden, indem Letztere eine Waschflüssigkeit zum Entfernen von Lösungsmittelresten zuführt. Danach kann der Formkörper 102 getrocknet werden.

Der Formkörper 102 kann darüber hinaus einer Nachbehandlung unterzogen werden, siehe die schematisch dargestellte Nachbehandlungseinheit 134. Zum Beispiel kann eine solche Nachbehandlung eine Hydroverschlingung, eine Nadelbehandlung, eine Imprägnierung, eine Dampfbehandlung mit einem unter Druck zugeführten Dampf und/oder ein Kalandrieren, etc., aufweisen.

Die Faseraufnahmeeinheit 132 kann den Formkörper 102 einer Aufwickeleinrichtung 136 zuführen, an welcher der Formkörper 102 aufgewickelt werden kann. Der Formkörper 102 kann dann als Rollgut einer Entität zugeführt werden, die Produkte wie zum Beispiel Wischtücher oder Textilien auf Basis des Formkörpers 102 herstellt.

Der Formkörper 102 kann dann direkt verwendet oder in einer in Figur 1 schematisch mit Bezugszeichen 162 dargestellten Weise zu einem bestimmten Produkt 150 (zum Beispiel einem Textilgewebe) weiterverarbeitet werden. Während der Lebensdauer eines solchen Produkts 150 kann es vorkommen, dass das Produkt 150 nachverfolgt werden soll, dessen Originalität bzw. Herkunft angezweifelt wird oder ermittelt bzw. überprüft werden soll. Im Weiteren wird ein System und ein Verfahren zum Identifizieren eines solchen Cellulose aufweisenden Produkts 150 gemäß einem exemplarischen Ausführungsbeispiel der Erfindung beschrieben.

Zunächst kann optional während dem Herstellen des Produkts 150 (genauer gesagt nach dem Koagulieren der Cellulose aufweisenden Filamente zum Bilden der Cellulosefasern 108 aus der Spinnlösung 104), das Produkt 150 an einer von der Steuereinheit 140 gesteuerten Markiereinrichtung 164 mit einem extrinsischen Bestandteil 160 versetzt werden, zum Beispiel ein Luminophor.

Das mit dem extrinsischen Bestandteil 160 versetzte Produkt 150 oder (wie in Figur 1 dargestellt) eine Vorform des Produkts 150 in Form des Formkörpers 102) kann in einer Messeinheit 166 vermessen werden. Zum Beispiel kann die Messeinheit 166 ein Spektrometer (beispielsweise ein Massenspektrometer, ein NMR-Spektrometer, etc.) aufweisen, mit dem das Produkt 150 oder dessen Vorform in Form des Formkörpers 102 hinsichtlich seiner bzw. ihrer Zusammensetzung aus mehreren Substanzen messtechnisch untersucht und charakterisiert wird.

In der Messeinheit 166 kann das Produkt 150 oder seine Vorform also hinsichtlich einer Kombination von in dem Produkt 150 oder in der Vorform vorhandenen Konstituenten bzw. Bestandteilen hinsichtlich Art und Menge vermessen werden. Die untersuchten Bestandteile können ganz oder teilweise nicht-cellulosische Bestandteile des Produkts 150 oder des Formkörpers 102 sein. Als qualitativ oder quantitativ analysierte nicht-cellulosische Bestandteile des Produkts 150 oder seiner Vorform können einzelne Spurenelemente, insbesondere Metalle, weiter insbesondere Seltenerdmetalle, Metalloxide wie Titandioxid bzw. Zinkoxid, ein Farbstoff (zum Beispiel Indigo aus Denim-Produkten), ein Leuchtstoff und synthetischer Kunststoff wie Elastan bzw. Polyester untersucht werden. Solche nicht-cellulosischen Bestandteile können aus Post-Consumer-Alttextilien stammen, die während des oben beschriebenen Herstellungsverfahrens in Form von Cellulosequelle 154 als Teil des Ausgangsstoffs 110 verwendet wurden. Solche Alttextilien enthalten häufig die genannten und andere Fremdstoffe, die sich dann auch in dem Produkt 150 bzw. dem Formkörper 102 wiederfinden.

Auch eine (oder mehrere) weitere Cellulosequelle 156, die zusätzlich zu den Alttextilien als Teil des Ausgangsstoffs 110 zugeführt werden kann, kann solche Fremdstoffe beinhalten. Somit können sich die Bestandteile, die mittels der Messeinheit 166 in dem Produkt 150 oder dessen Vorform in Form des Formkörpers 102 vermessen werden, auf intrinsische Bestandteile des Produkts 150 oder der Vorform beziehen, die bereits in dem Ausgangsstoff 110 und somit in den gebildeten Cellulosefasern 108 enthalten waren.

Auch mindestens ein cellulosischer Bestandteil des Produkts 150 oder seiner Vorform kann von der Messeinheit 166 vermessen werden, um das Produkt 150 bzw. seine Vorform in Form des Formkörpers 102 zu charakterisieren.

Ferner kann ergänzend der optionale extrinsische Bestandteil 160 von der Messeinheit 166 gemessen werden.

Als Ergebnis der Messung der Messeinheit 166 an einem bestimmten Produkt 150 bzw. seiner Vorform in Form des Formkörpers 102 kann ein mit dem Produkt 150 bzw. seiner Vorform korrelierter Datensatz 180 in einer Datenbank 152 gespeichert werden. Die Datenbank 152 kann Datensätze 180 sowie andere Daten aufweisen, die zum Beispiel auf einem elektronischen Massenspeicher oder einem Teil eines elektronischen Massenspeichers, wie zum Beispiel eine Festplatte eines Computers, gespeichert sein können. In dieser Datenbank 152 werden eine Vielzahl von Datensätzen 180 gespeichert, die ein jeweiliges Produkt 150 bzw. seine Vorform mit den zugehörigen Messergebnissen der Messeinheit 166 korrelieren. Jeder der Datensätze 180 enthält für ein zugehöriges Produkt 150 bzw. dessen Vorform die Information hinsichtlich der intrinsischen bzw. extrinsischen nicht-cellulosischen bzw. cellulosischen Bestandteile. Es ist ebenfalls möglich, dass der Datensatz 180 Informationen hinsichtlich der Erscheinungsform der Cellulosefasern 108 (mithin Information über einen cellulosischen Bestandteil) enthält, die in dem Formkörper 102 bzw. in dem Produkt 150 enthalten sind. Dies korrespondiert, wie bezugnehmend auf Figur 3 bis Figur 5 erläutert wird, Rückschlüsse zu einem verwendeten Verfahren zum Herstellen der Cellulosefasern 108 (Lyocell-Verfahren oder Viskoseverfahren).

Messtechnisch erfasste Information bzw. daraus abgeleitete Information kann zum Beispiel in Form von qualitativen (Bestandteil vorhanden oder nicht vorhanden) oder quantitativen (Bestandteil mindestens oder höchstens in einer gewissen Menge vorhanden) Daten in den jeweiligen Datensatz 180 enthalten sein. Alternativ oder ergänzend kann entsprechende Information aber auch in Form von logischen Werten hinsichtlich Kriterien enthalten sein, die sich auf die genannten Bestandteile beziehen (zum Beispiel einem logischen Wert "0", wenn ein Bestandteil in einem Produkt 150 oder dessen Vorform mindestens mit einer vorgegebenen Menge enthalten war oder einem logischen Wert "1", wenn ein Bestandteil in einem Produkt 150 oder dessen Vorform nicht mindestens mit einer vorgegebenen Menge enthalten war, oder umgekehrt). Es ist also möglich, die genannte Information in dem Datensatz 180 in Form von Bits, d.h. in binärer Logik, abzuspeichern. Insbesondere kann dadurch der ermittelte Datensatz 180 dafür indikativ sein, ob das Produkt 150 zumindest teilweise auf Basis einer wiederverwerteten Cellulosequelle 154 in Form der Alttextilien als zumindest Teil des Ausgangsstoffs 110 hergestellt wurde.

Die genannte Information, die in den jeweiligen Datensatz 180 eingeht, kann auf Basis der Messwerte der Messeinheit 166 von einer Prozessoreinheit 170 bestimmt werden. Diese Prozessoreinheit 170 kann optional mit der Steuereinheit 140 gekoppelt sein oder kann Teil derselben bilden.

Wie in Figur 1 schematisch dargestellt, kann in der Datenbank 152 über den Datensatz 180 für das Produkt 150 "P1" hinaus als weiterer Datensatz 180 für ein weiteres Produkt 151 "P2" in entsprechender Weise auch für das weitere Produkt 151 ein damit korrelierter Datensatz 180 gespeichert werden. Auf diese Weise kann die Datenbank 152 hinsichtlich vieler unterschiedlicher Produkte 150, 151, ... einen zugehörigen Datensatz 180 enthalten. Alternativ ist es auch möglich, dass ein jeweiliger Datensatz 180 in der Datenbank 152 allen Produkten 150 einer gemeinsamen Produktionscharge zugeordnet ist und dass andere Produkte 151 anderer Produktionschargen mit jeweils anderen Datensätzen 180 in der Datenbank 152 korreliert sind.

Möchte ein Benutzer für ein Testprodukt 158 (oder für eine Vorform desselben) zum Beispiel für Nachverfolgungszwecke oder als Ursprungsnachweis wissen, welche Identität dieses Testprodukt 158 hat oder in welcher Charge dieses Testprodukt 158 produziert wurde, kann der Benutzer ein solches Testprodukt 158 mit einem Prüfgerät 174 vermessen. Das Prüfgerät 174 kann zum Beispiel als portables Gerät ausgebildet sein. Das Prüfgerät 174 kann eine Messeinrichtung 176 zum Messen der Information hinsichtlich der Konstituenten des Testprodukts 158 aufweisen, in gleicher oder ähnlicher Weise wie die Messeinheit 166. Das Prüfgerät 174 ist also zum Ermitteln von Information eingerichtet, die für mehrere Bestandteile des Testprodukts 158 indikativ ist. Eine Verarbeitungseinrichtung 178 des Prüfgeräts 174 kann die von der Messeinrichtung 176 messtechnisch erfasste Information verarbeiten. Insbesondere kann die Verarbeitungseinrichtung 178 im Zusammenwirken mit einer Kommunikationseinrichtung 186 des Prüfgeräts 174 aus der Datenbank 152 Datensätze 180 abfragen, indem die Kommunikationseinrichtung 186 des Prüfgeräts 174 mit einer mit der Datenbank 152 verbundenen anderen Kommunikationseinrichtung 188 über ein Kommunikationsnetzwerk 190 (zum Beispiel das öffentliche Internet) kommuniziert und dabei Daten überträgt.

Hat die Verarbeitungseinrichtung 178 des Prüfgeräts 164 Zugang zu den Datensätzen 180 aus der Datenbank 152 erlangt, wird die von der Messeinrichtung 176 erfasste Information mit den Datensätzen 180 in der Datenbank 152 abgeglichen. Hierbei wird zum Beispiel derjenige Datensatz 180 aus der Datenbank 152 ermittelt, der die bestmögliche Übereinstimmung mit den von der Messeinrichtung 176 für das Testprodukt 158 ermittelten Informationen hinsichtlich dessen Konstituenten zeigt. Zum Beispiel kann also ein Identifizieren des Testprodukts 158 als einem der Datensätze 180 zugehörig erfolgen, wenn eine Diskrepanz zwischen der von der Messeinrichtung 176 erfassten Information hinsichtlich des Testprodukts 158 einerseits und einem der Datensätze 180 andererseits einen vorgegebenen Schwellwert unterschreitet oder nicht überschreitet. Als geeigneter Schwellwert kann insbesondere eine Anzahl von erfüllten bzw. nicht erfüllten Bitkriterien 184 verwendet werden, vergleiche Figur 2. Das Produkt 150, 151, ..., das diesem am besten passenden Datensatz 180 zugeordnet ist, kann als identisch zu dem Testprodukt 158 (oder zumindest als derselben Produktionscharge zugehörig) identifiziert werden. Auf diese Weise kann die Originalität des Testprodukts 158 verifiziert und dessen Zuordnung zu einer Produktionscharge ermöglicht werden. Schlägt diese Identifizierung fehlt, kann die Schlussfolgerung gezogen werden, dass das Testprodukt 158 nicht aus der Apparatur 100 stammt und möglicherweise eine Fälschung darstellt.

Das Ergebnis dieser Analyse kann einem Benutzer an einer Anzeigeeinrichtung 199 des Prüfgerätes 174 angezeigt werden. Gegebenenfalls kann das Ergebnis dieser Analyse auch an die Prozessoreinheit 170 über das Kommunikationsnetzwerk 190 übermittelt werden.

**Figur 2** zeigt eine Bitfolge 182 eines Datensatzes 180, der ein Produkt 150 mit einer mittels eines Lyocell-Verfahrens hergestellten Cellulosefaser 108 identifiziert. Gemäß Figur 2 wird also, wie bereits bezugnehmend auf Figur 1 beschrieben, als Datensatz 180 eine für eine Zusammensetzung des Produkts 150 aus den diversen Bestandteilen indikative Bitfolge 182 in der Datenbank 152 hinterlegt bzw. gespeichert.

Figur 2 zeigt also, wie insgesamt 14 Bits einem bestimmten Produkt 150 (bzw. einer zugehörigen Vorform in Form des Formkörpers 102) als Datensatz 180 zugeordnet sind. Sechs Bits P0 bis P5 beziehen sich auf bestimmte Kriterien, welche das Produkt 150 oder deren Vorform erfüllt oder nicht. In dem dargestellten Ausführungsbeispiel ist zum Beispiel ein Titandioxidkriterium (d.h. ein Kriterium betreffend eine Menge des Bestandteils TiO₂) in dem Produkt 150 erfüllt, sodass das zugehörige Bit den logischen Wert "1" hat. Dagegen ist ein Kriterium hinsichtlich Eisenoxid Fe₂O₃ bei dem Produkt 150 nicht erfüllt, sodass das zugehörige Bit den logischen Wert "0" hat. Acht weitere Bits S0 bis S7 beziehen sich auf weitere luminophorbasierte Kriterien, welche das Produkt 150 oder deren Vorform ebenfalls zum Teil erfüllt und zu einem anderen Teil nicht erfüllt. Die Bitfolge 182 des Produkts 150 (01100001100000 im dargestellten Beispiel) ist also der Code für dieses Produkt 150 und ein charakteristischer Fingerabdruck des Produkts 150. Ein Testprodukt 158 kann als identisch zu diesem Produkt 150 angesehen werden, wenn für das Testprodukt 158 dieselbe Bitfolge (01100001100000 im dargestellten Beispiel) ermittelt wird.

Im Weiteren werden einige weitere Aspekte exemplarischer Ausführungsbeispiele der Erfindung beschrieben:
Gemäß einem exemplarischen Ausführungsbeispiel wird ein Codiersystem für Lyocellfasern mit Rezyklatanteil geschaffen. Fasern (insbesondere Cellulosefasern 108), welche nach dem Lyocell-Verfahren hergestellt wurden, können in den verschiedensten Anwendungen eingesetzt werden. Das Recycling von Materialien im Sinne eines möglichst geschlossenen Stoffkreislaufes ist ein Bedürfnis unter dem Gesichtspunkt von Ressourcenschonung, Nachhaltigkeit und Umweltschutz. Für Konsumenten kann ein überprüfbares Merkmal vorteilhaft sein, das den Rezyklatanteil eines Produkts 150 bestätigt, d.h. eine Überprüfung erlaubt, ob das Produkt 150 auf Basis wiederverwerteter Cellulosequellen 154, 156 hergestellt worden ist. Unter dem Begriff "Rezyklat" können im Rahmen der vorliegenden Anmeldung insbesondere aus dem Stoffkreislauf zurückgewonnene Materialien der Gattungen Garne, Textilien oder Vliese (d.h. Nonwovens) verstanden werden, welche (insbesondere frühestens) während der Zellstoffherstellung im Rahmen des Lyocell-Verfahrens oder bei der Garnherstellung mitverwendet werden.

Exemplarische Ausführungsbeispiele der Erfindung beruhen unter anderem auf der Erkenntnis, dass im Rahmen der Lyocellproduktion eine Identifikation des Rezyklats im Sinne von quantitativer Materialidentifikation vor oder während der Rezyklatverarbeitung wünschenswert bzw. vorteilhaft ist. Ausführungsbeispiele der Erfindung schaffen daher ein nur schwer fälschbares bzw. fälschungssicheres Markierungssystem zur Überprüfung, ob zumindest Anteile rezyklierter Materialien in einer Lyocellfaser eines Produkts 150 vorhanden sind. Insbesondere wird gemäß einem exemplarischen Ausführungsbeispiel ein Markierungssystem bereitgestellt, bei welchen verschiedene Inhaltsstoffe in einem Produkt 150 oder einer Vorform die einzelnen Bits einer Bitfolge 182 eines Codiersystemes zum Originalitätsnachweis des entsprechenden Produkts 150 abbilden. Mit Vorteil können solche Inhaltsstoffe bzw. Bestandteile einen direkten Bezug zum markierten Inhalt aufweisen, womit eine zusätzliche Selbstmarkierung erreicht wird.

Durch Inhalts- oder Zusatzstoffe, für welche sichergestellt ist, dass sie im Dope bzw. einer Spinnlösung 104 eines Lyocellprozesses in geeigneter Konzentration vorhanden sind, kann ein Markersystem gebildet werden, das eine binäre Codierung erlaubt (vergleiche Figur 2). Durch das Erzeugen eines Lyocell-Cellulose aufweisenden Formkörpers 102 (zum Beispiel in Form einer Faser) können diese Markerbestandteile im Inneren der Formkörpers 102 eingebettet werden und so mit hoher Fälschungssicherheit verankert werden.

Eine zusätzliche Fälschungsresistenz kann gemäß exemplarischen Ausführungsbeispielen der Erfindung durch Markerbits erreicht werden, die eine Teileigenschaft der nachzuweisenden Originalität sind. So ist zum Beispiel das gezielte Einhalten von Anteilen einer Baumwollfaser in einem Rezyklat ein aufwendigerer Prozess als einfach unkontrolliert alles Rezyklat zu verwenden. Solche anteils- oder eigenschaftsbezogenen Markerbits an der nachzuweisenden Eigenschaft im Endprodukt in Kombination mit Markerbits in Bezug auf ein qualitatives oder sogar quantitatives Vorkommen von Bestandteilen in einem Produkt 150 (zum Beispiel eine Bitwertbasis aufgrund des Nachweises von Stoffen oder Verbindungen mit minimalen Anteilen von Seltenen Erden, etc.) bilden zusammen eine Bitfolge 182, die auch als Superbitsequenz bezeichnet werden kann. Eine solche Superbitsequenz kann auch die Codierung von Nebeninformationen (wie zum Beispiel Herstellungsdatum, Charge, Hersteller, Identifizierer eines Produkts 150, etc.) nebst ebenfalls über einen anderen Bereich der Bitfolge 182 bzw. Bitsets verankerten Originaltitätsentitäten des Produkts 150.

Gemäß einem Ausführungsbeispiel kann die Bitfolge 182 ein oder vorzugsweise mehrere primäre Markerbits 192 mit inhaltlichem und/oder funktionalem und/oder stofflichem Bezug zu einem Lyocell-basierten Produkt 150 und ein oder vorzugsweise mehrere sekundäre Markerbits 194 betreffend Materialien mit reinem Markercharakter aufweisen. Ein solches auch in Figur 2 dargestelltes Superbitset setzt sich also vorzugsweise aus sekundären Markerbits 194 von Materialien mit reinem Markercharakter (zum Beispiel Luminophore) und aus primären Markerbits 192 zusammen, welche einen inhaltlichen und/oder funktionalen und/oder stofflichen Bezug zum lyocellbasierenden Spezialprodukt (zum Beispiel Rezyklatspezialisierung, Naturfasern (zum Beispiel Baumwollfasern), optische Aufheller, Kunststoffe, fluoreszierende Komponenten) haben können. Dabei kann die Konzentration der primären Markerbits 192 derart gesteuert werden, dass sie einer typischen Konzentration des entsprechenden lyocellbasierenden Spezialproduktes entsprechen. Dies bedeutet in dem beschriebenen Beispiel, dass das Bitset der primären Markerbits 192 zur Sicherstellung der zusätzlichen Fälschungsresistenz vorzugsweise nicht nur "Null" sein sollte ("Null" bedeutet, dass die Bitfolge 182 bzw. das Bitset ohne alle Markerbits gesetzt ist). Das Bitset der sekundären Markerbits 194 kann demgegenüber ein leeres Bitset sein, so dass der ganze mögliche Umfang des binären Repräsentationsbereiches dieses Sets von sekundären Markern 194 ausgeschöpft werden kann.

Durch die Möglichkeit des unabhängigen Vorhandenseins der einzelnen primären Markerbits 192 kann sichergestellt werden, dass durch das Set von primären Markerbits 192 alle möglichen Kombinationen in einem Lyocell-Produkt 150 codiert und somit abgebildet werden können. Das folgende Beispiel illustriert diese kombinatorischen Möglichkeiten: Wenn ein Bit, das durch das Vorhandensein einer gewissen Konzentration von Farbstoffresten definiert ist, und ein weiteres Bit betrachtet werden, das durch das Vorhandensein von einer bestimmten Konzentration von Baumwollfasern definiert ist, lassen sich folgende vier Zustände codieren:

| Farbstoffbit | Baumwollbit | Codierung |
|---|---|---|
| 0 | 0 | Uncodiertes normales Lyocellprodukt |
| 1 | 0 | Lyocellprodukt, mit Rezyklat, das schon mal gefärbt war |
| 0 | 1 | Lyocellprodukt, mit Rezyklat aus Baumwolle |
| 1 | 1 | Lyocellprodukt, mit Rezyklat aus Baumwolle, die gefärbt war |

Durch die zusätzlichen sekundären Markerbits 194 können unabhängig von der Produktzusammensetzung verschiedene weitere Produktdetails codiert werden. So kann beispielshaft durch die folgenden fünf sekundären Markerbits 194 (SM0 bis SM4) folgende Zusatzinformation codiert werden:

| SM0 | SM1 | SM2 | SM3 | SM4 | Bedeutung |
|---|---|---|---|---|---|
| 0 | 0 | x | x | x | Hersteller = v |
| 0 | 1 | x | x | x | Hersteller = w |
| 1 | 0 | x | x | x | Hersteller = y |
| 1 | 1 | x | x | x | Hersteller = z |
| x | x | 0 | 0 | 0 | Herstellungsjahr nicht codiert |
| x | x | 0 | 0 | 1 | Herstellungsjahr 2018 |
| x | x | 0 | 1 | 0 | Herstellungsjahr 2019 |
| x | x | 1 | 0 | 0 | Herstellungsjahr 2020 |

Eine Faser als Formkörper 102 bzw. ein Produkt 150 mit der Kombination SM0=0, SM1=1, SM2=0, SM3=1, SM4=0 von sekundären Markerbits 194 kann in diesem Beispiel bedeuten: Vom Hersteller "w" im Jahr 2019 hergestellt.

In Kombination mit dem Set von primären Markerbits 192 gemäß vorhergehender Darstellung (Baumwollbit=0, Farbstoffbit=l) kann zusammen mit dem Set von sekundären Markerbits 194 SM0=0, SM1=1, SM2=0, SM3=1, SM4=0 dann bedeuten: Vom Hersteller "w" im Jahr 2019 hergestellte Lyocellfaser mit einem Anteil von Rezyklat, das aus gefärbten Fasern gewonnen wurde.

Die sekundären Marker 194 können gemäß einem exemplarischen Ausführungsbeispiel insbesondere aus einem Leuchtstoff bestehen (zum Beispiel als eine spezialisierte Form der Markierung mit Seltenen Erden). Anorganische Leuchtstoffe können geeignet sein, direkt durch einen Konsumenten am Ort des Kaufs eines Produkts 150 eine Überprüfung auf die Originalität eines Rezyklatsproduktes hin durchzuführen.

Bestimmte Stoffe bzw. Verbindungen können sowohl als primäre Marker 192 oder als sekundäre Marker 194 zur Anwendung kommen. Zum Beispiel sind fluoreszente Leuchtstoffe als Originalitätsmarker verwendbar. Optische Aufheller, welche einen steuerbaren oder selektierbaren Anteil eines Rezyklates ausmachen, haben aber sehr ähnliche optische bzw. physikalische Eigenschaften. So kann je nach Detailfunktionalisierung, Dosierung und Nebeneigenschaften ein solcher Leuchtstoff gemäß einem exemplarischen Ausführungsbeispiel der Erfindung als primärer Marker 192 oder als sekundärer Marker 194 eingesetzt werden.

Während die primären Marker 192 unabhängige Einzelbits darstellen können (also bedeutungsmäßig orthogonal sein können), können die sekundären Marker 194 je nach Codierung mehrere, aber zum Beispiel mindestens eine Dualzahl bilden. Eine solche Dualzahl kann eine individuell festgelegte Bedeutung bzw. Codierung haben, welche zum Beispiel nur produktionsintern verwendet wird. Andererseits kann diese Codierung auch an einer Hinterlegungsstelle hinterlegt werden, um zum Beispiel in einem Streitfall die Originalität des Produktes 150 zu sichern, so dass der Endkunde selbst eine Überprüfung vornehmen kann. Die generische Darstellung der Kombination von primären Markerbits 192 und sekundären Markerbits 194 zu einem Superbitset stellt sich zum Beispiel in der in Figur 2 gezeigten Weise dar.

Gemäß einem exemplarischen Ausführungsbeispiel der Erfindung können durch eine Durchmischung in der Spinnlösung 104 bzw. im Dope, die Markermaterialien in einer homogenen Verteilung (und bevorzugt fein dispergiert) gehalten werden, so dass für die Nachweisbarkeit der primären Markerbits 192 und der sekundären Markerbits 194 eine gleichmäßige Verteilung über große Fasermengen bzw. Produktmengen entsteht. Insbesondere erlaubt eine solche homogene Verteilung auch eine sehr niedrige Dosierung für gewisse Markermaterialien (zum Beispiel Spurenelemente, insbesondere Metalle, weiter insbesondere Seltenerdmetalle). Insbesondere für die Verwendung von Markermaterialien in nanoskaliger Größe ist es vorteilhaft, eine hohe Dispergierung einzustellen. Dies kann zum Beispiel durch Beaufschlagung mit Ultraschall und/oder mit Mikrowellen erreicht oder zumindest unterstützt werden.

Gemäß einem exemplarischen Ausführungsbeispiel der Erfindung wird das beschriebene Markierungsprinzip im großtechnischen Maßstab eingesetzt. Durch die Analyse der primären Markerbits 192 im Rezyklat kann so der Faktor des Textil-Stoffkreislaufes ermittelt werden. Dieser Faktor kann wiederum zur Steuerung des Rezyklatanteils und der Rezyklatzusammensetzung verwendet werden. Aufgrund Degradierung und einer folglichen Reduzierung des durchschnittlichen Polymerisationsgrads kann eine Cellulosefaser 108 mehrere Male (zum Beispiel sechsmal) den Stoffkreislauf eines Lyocell-Verfahrens durchlaufen, ohne ihre Recyclingfähigkeit zu verlieren. Der über die primären und sekundären Markerbits 192, 194 codierte Lyocell-Formkörper 102 erlaubt die Durchführung der entsprechenden Mess- und Steuerverfahren zur Abschätzung der Textil-Stoffkreisläufe und der Steuerung der Rezyklatqualität eines der Lyocellproduktion zugefügten Rezyklates.

In einem anderen exemplarischen Ausführungsbeispiel der Erfindung werden dem Dope bzw. der Spinnlösung 104 ein oder mehrere Kontrast- oder Indikationsmittel zugegeben, welche in der resultierenden Cellulosefaser 108 nachgewiesen werden können. Dabei können die unterschiedlichen Zusatzstoffe wiederum als einzelne Bits des Superbitsets in die Codierung einbezogen werden. Die gemäß dem beschriebenen Ausführungsbeispiel eingesetzten Kontrast- und Indikationsmittel können mittels unterschiedlicher Nachweisverfahren bestimmt werden. Dazu gehört die Kernspinresonanzspektroskopie (NMR-Spektroskopie), mikroskopische Verfahren, insbesondere Fluoreszenzmessverfahren, Röntgenverfahren, Infrarot- und Raman-Spektroskopie, sowie die Photoelektronenspektroskopie (PES), insbesondere Röntgen- und Ultraviolet-PES (XPS bzw. UPS). Auch Verfahren zur Messung einer Molekulargewichtverteilung und/oder extrahierbaren Inhaltsstoffen des Produkts 150 oder des Formkörpers 102 können diesbezüglich Einsatz finden. Entsprechendes gilt für Verfahren zum Nachweis von Farbstoffresten in dem Produkt 150 oder seiner Vorform. Kontrast- oder Identifikationsmittel können wegen ihrer Unabhängigkeit als sekundäre Markerbits 194 eingesetzt werden. Andere Kontrast- oder Identifikationsmittel können eine Funktion oder ein Resultat in der Zellulosefaser 108 mitbegründen und können daher mit Vorteil als primäre Markerbits 192 eingesetzt werden. Diese Materialien werden bevorzugt in nanoskaligem Maßstab und niedrigen Konzentrationen verwendet. Mit Vorteil kann eines oder können mehrere der folgenden Materialien eingesetzt werden:

| Materialgruppe | Primäre/sekundäre Markerbits |
|---|---|
| Partikel mit Fluoreszenz- und/oder Phosphoreszenzeigenschaften | Primär und Sekundär |
| kombinierte, multimodale Systeme zur Signalgebung | Sekundär |
| Pigmente aus Rezyklaten | Primär |
| Maqnetkontrastmittel | Primär |
| Röntqenkontrastmittel | Sekundär |
| Anorganische Mikro- und/oder Nanopartikel | Sekundär |
| Formselektierte Partikel | |

In einer bevorzugten Ausführungsform werden mindestens zwei primäre Marker verwendet, die mindestens zwei primären Markerbits 192 zugeordnet sind. Durch die Messung des Verhältnisses der Markermengen zueinander lassen sich Rückschlüsse auf die Verdünnung im Stoffkreislauf bzw. auf Anreicherungen von Fremdmaterialien während der Eingangskontrolle des Rezyklats vor dem Lyocellprozess bilden.

In einer weiteren bevorzugten Ausführungsform werden geringe Mengen eines Leuchtstoffmarkers beigefügt. Damit diese weniger durch den Lyocell-, oder Viskoseprozess chemisch angegriffen werden, können diese vor der Zugabe in das Dope bzw. die Spinnlösung 104 mittels einer (zum Beispiel nanoskaligen glasartigen) Hülle umgeben werden (zum Beispiel mittels eines Sol-Gel-Prozesses). Durch die Einbettung direkt ins Dope bzw. in die Spinnenlösung 104 kann eine hohe Auswaschsicherheit und somit eine hohe Fälschungssicherheit im Produkt erreicht werden. Bei einer geeigneten Dosierung dieses sekundären Markers kann zur direkten Identifikation des Rezyklatproduktes durch den Kunden mittels eines stationären spektroskopischen Analysators die Authentizität der Rezyklatverwendung direkt durch den Kunden selbst festgestellt werden. Eine optionale Zusatzvariante besteht in der Realisierung einer App auf einem mobilen Gerät (zum Beispiel ein Mobilfunkgerät wie ein Smartphone), das in der Lage ist, die erforderlichen spektroskopischen Auswertungen durchzuführen. So kann der Kunde selbst ohne Unterstützung die Echtheit von Produkten 150 überprüfen, für welche ein Rezyklatanteil oder ein geschlossener Stoffkreislauf beansprucht wird.

Eine nach dem Lyocell-Verfahren hergestellter Lyocell-Formkörper 102 kann gemäß einem exemplarischen Ausführungsbeispiel der Erfindung einen Rezyklatanteil (insbesondere einen Anteil von Altkleidern im Ausgangsstoff 110) von mindestens 3 Gewichtsprozent aufweisen und mindestens einen intrinsischen Bestandteil zur Verwendung als Marker mit folgenden Markerkriterien aufweisen:
a) Einen Anteil von Seltenerdenelementen von mindestens 1 ppm, insbesondere von mindestens 5 ppm
b) Einen Anteil von TiO₂ von mindestens 5 ppm, insbesondere von mindestens 10 ppm
c) Einen Anteil von Restbestandteilen von Farbstoffen von mindestens 10 ppm, insbesondere von mindestens 20 ppm
d) Einen Anteil von Metalloxyden von vorzugsweise mindestens 50 ppm
e) Einen Anteil eines formselektierten Partikels (zum Beispiel ein sphärisches Partikel) von mindestens 1 Partikel pro mm³ Formkörpervolumen, bevorzugt mehr als 10 Partikel pro mm³ Formkörpervolumen

Der Lyocell-Formkörper 102 kann mit Vorteil einen Leuchtstoff, bevorzugt einen anorganischen Leuchtstoff, enthalten.

Der Lyocell-Formkörper 102 bzw. das Produkt 150 kann mittels eines spektroskopischen Verfahrens auf primäre Marker und/oder sekundäre Marker überprüft werden. Dieses spektroskopische Verfahren kann zum Beispiel im Rahmen eines Verkaufsprozesses des Produkts 150 angewendet werden. Dies kann für einzelne Marker oder für alle Marker erfolgen.

Der Lyocell-Formkörper 102 bzw. das Produkt 150 kann also mittels eines vorzugsweise mobilen Gerätes auf primäre Marker und/oder sekundäre Marker überprüft werden. Dies kann für einzelne Marker oder für alle Marker erfolgen.

**Figur 3** zeigt eine mittels eines Lyocell-Verfahrens hergestellte Cellulosefaser 200 im Querschnitt. Die mittels eines Lyocell-Verfahrens hergestellte Cellulosefaser 200 hat eine glatte runde Außenfläche 202 und ist homogen und frei von makroskopischen Löchern mit Cellulosematerial ausgefüllt. Sie kann daher von einem Fachmann eindeutig von mittels eines Viskoseverfahrens hergestellten Cellulosefasern (siehe Bezugszeichen 204 in Figur 4) und von Cellulosefasern aus Baumwollpflanzen (siehe Bezugszeichen 206 in Figur 5) unterschieden werden.

**Figur 4** zeigt eine mittels eines Viskoseverfahrens hergestellte Cellulosefaser 204 im Querschnitt. Die Cellulosefaser 204 ist wolkenförmig und weist entlang ihres äußeren Umfangs eine Mehrzahl von bogenförmigen Strukturen 208 auf.

**Figur 5** zeigt eine natürliche Cellulosefaser 206 aus einer Baumwollpflanze im Querschnitt. Die Cellulosefaser 206 ist nierenförmig und weist im Inneren ein materialfreies Lumen 210 als vollumfänglich umschlossenen Hohlraum auf.

Anhand der signifikanten geometrischen bzw. strukturellen Unterschiede der Fasern gemäß Figur 3 bis Figur 5 ist es einem Fachmann möglich, zum Beispiel unter einem Mikroskop unzweideutig zu ermitteln, ob eine Cellulosefaser mittels des Lyocell-Verfahrens, mittels des Viskoseverfahrens oder natürlich in einer Baumwollpflanze gebildet worden ist.

Anhand der bezugnehmend auf Figur 3 bis Figur 5 beschriebenen Phänomene kann auch eine (zum Beispiel unter dem Mikroskop ermittelte) Form der Cellulosefasern 108 in einem Produkt 150 bzw. einer Vorform desselben (beispielsweise eines Formkörpers 102) herangezogen werden, um ein Testprodukt 158 zu identifizieren.

Ergänzend ist darauf hinzuweisen, dass "aufweisend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Verfahren zum Identifizieren eines Cellulose aufweisenden Produkts (150), wobei das Verfahren aufweist:
Ermitteln eines Datensatzes (180), der für mehrere Bestandteile des Produkts (150) oder einer Vorform des Produkts (150) indikativ ist;
Hinterlegen des mit dem Produkt (150) oder der Vorform korrelierten Datensatzes (180) in einer Datenbank (152).

2. Verfahren gemäß Anspruch 1, aufweisend zumindest eines der folgenden Merkmale:
wobei der ermittelte Datensatz (180) dafür indikativ ist, ob das Produkt (150) oder die Vorform auf Basis einer wiederverwerteten Cellulosequelle (154) als zumindest Teil eines Ausgangsstoffs (110) hergestellt wurde;
wobei das Produkt (150) oder die Vorform auf Basis von Altkleidern als Cellulosequelle (154) als zumindest Teil eines Ausgangsstoffs (110) hergestellt wird;
wobei das Produkt (150) oder die Vorform auf Basis von Resten aus einer Kleidungsherstellung als Cellulosequelle (154) als zumindest Teil eines Ausgangsstoffs (110) hergestellt wird.

3. Verfahren gemäß Anspruch 2, wobei das Produkt (150) oder die Vorform zumindest teilweise auf Basis von einer weiteren Cellulosequelle (156) hergestellt wird, insbesondere auf Basis von zumindest einem Material aus einer Gruppe, die besteht aus Holzzellstoff, Hadernzellstoff, Baumwolle, mittels eines Lyocell-Verfahrens hergestellte Cellulose und mittels eines Viskoseverfahrens hergestellte Cellulose.

4. Verfahren gemäß einen der Ansprüche 1 bis 3, aufweisend zumindest eines der folgenden Merkmale:
wobei das Produkt (150) oder die Vorform auf Basis eines Ausgangsstoffs (110) hergestellt wird, der als inhomogene Mischung unterschiedlicher Bestandteile hergestellt wird;
wobei das Produkt (150) oder die Vorform mittels des Lyocell-Verfahrens oder mittels des Viskoseverfahrens hergestellt wird.

5. Verfahren gemäß einem der Ansprüche 1 bis 4, wobei das Verfahren aufweist:
Analysieren eines zu identifizierenden Testprodukts (158) zum Ermitteln von Information, die für mehrere Bestandteile des Testprodukts (158) indikativ ist;
Abgleichen der Information mit dem Datensatz (180) in der Datenbank (152).

6. Verfahren nach einem der Ansprüche 1 bis 5, wobei das Verfahren ferner aufweist:
Ermitteln mindestens eines anderen Datensatzes (180), der für mehrere Bestandteile mindestens eines anderen Produkts (151) oder einer Vorform des mindestens einen anderen Produkts (151) indikativ ist;
Hinterlegen des mit dem mindestens einen anderen Produkts (151) oder seiner Vorform korrelierten Datensatzes (180) in der Datenbank (152).

7. Verfahren gemäß Ansprüchen 5 und 6, wobei das Verfahren aufweist:
Abgleichen der Information mit dem mindestens einen anderen Datensatz (180); und
Identifizieren des Testprodukts (158) als einem des Datensatzes (180) und dem mindestens einen anderen Datensatz (180) zugehörig, wenn:
die Information und dieser Datensatz (180) eine bestmögliche Übereinstimmung zeigen, insbesondere eine höchste Anzahl von übereinstimmenden Bitkriterien (184) hinsichtlich einer für eine Zusammensetzung des Produkts (150) oder der Vorform aus den mindestens zwei Bestandteilen indikativen Bitfolge (182) zeigen; und/oder
eine Diskrepanz zwischen der Information einerseits und einem von dem Datensatz (180) und dem mindestens einen anderen Datensatz (180) andererseits einen vorgegebenen Schwellwert, insbesondere eine Anzahl von nicht erfüllten Bitkriterien (184) hinsichtlich einer für eine Zusammensetzung des Produkts (150) oder der Vorform aus den mindestens zwei Bestandteilen indikativen Bitfolge (182), unterschreitet oder nicht überschreitet.

8. Verfahren gemäß einem der Ansprüche 1 bis 7, aufweisend zumindest eines der folgenden Merkmale:
wobei der Datensatz (180) für eine Kombination von in dem Produkt (150) vorhandenen Bestandteilen, insbesondere für eine Kombination von in dem Produkt (150) in einer jeweiligen Menge enthaltenen Bestandteilen, indikativ ist;
wobei sich die mehreren Bestandteile, insbesondere ausschließlich, auf nicht-cellulosische Bestandteile des Produkts (150) oder der Vorform beziehen;
wobei sich mindestens einer der mehreren Bestandteile auf mindestens einen cellulosischen Bestandteil des Produkts (150) oder der Vorform bezieht;
wobei das Verfahren während oder nach einem Herstellen des Produkts (150) oder der Vorform, insbesondere nach einem Koagulieren von Cellulose aufweisenden Filamenten aus einer Spinnlösung (104), ein Versetzen des Produkts (150) oder der Vorform mit mindestens einem extrinsischen Bestandteil (160) aufweist, für den der in der Datenbank (152) hinterlegte Datensatz (180) ebenfalls indikativ ist;
wobei der Datensatz (180) einer Produktionscharge des Produkts (150) zugeordnet wird.

9. Verfahren gemäß einem der Ansprüche 1 bis 8, wobei bei dem Verfahren als Datensatz (180) eine für eine Zusammensetzung des Produkts (150) oder der Vorform aus den mindestens zwei Bestandteilen indikative Bitfolge (182) in der Datenbank (152) hinterlegt wird.

10. Verfahren gemäß Anspruch 9, wobei die Bitfolge (182) aufweist:
mindestens ein primäres Markerbit (192) mit inhaltlichem und/oder funktionalem und/oder stofflichem Bezug zu dem, insbesondere Lyocell-basierten, Produkt (150) oder der, insbesondere Lyocell-basierten, Vorform; und
mindestens ein sekundäres Markerbit (194) betreffend Materialien des, insbesondere Lyocell-basierten, Produkts (150) oder der, insbesondere Lyocell-basierten, Vorform mit reinem Markercharakter, insbesondere ohne inhaltlichen und/oder ohne funktionalen und/oder ohne stofflichen Bezug zu dem, insbesondere Lyocell-basierten, Produkt (150) oder der, insbesondere Lyocell-basierten, Vorform.

11. Verfahren gemäß einen der Ansprüche 1 bis 10, wobei das Produkt (150) oder die Vorform Formkörper (102) aufweist.

12. Verfahren gemäß Anspruch 11, wobei die Formkörper (102) regenerierte cellulosische Formkörper (102) sind, insbesondere Fasern, Folien, Schwämme oder Kugeln sind.

13. Verfahren gemäß einem der Ansprüche 1 bis 12, wobei sich die mehreren Bestandteile, insbesondere ausschließlich, auf intrinsische Bestandteile des Produkts (150) oder der Vorform beziehen, die bereits nach einer Extrusion einer Spinnlösung (104) zu cellulosischen Formkörpern (102) in und/oder an den Formkörpern (102) enthalten waren.

14. Verfahren gemäß einem der Ansprüche 8 bis 13, aufweisend zumindest eines der folgenden Merkmale:
wobei die intrinsischen Bestandteile und/oder die nicht-cellulosischen Bestandteile zumindest einen aus einer Gruppe aufweisen, die besteht aus einem Spurenelement, insbesondere einem Metall, weiter insbesondere einem Seltenerdmetall, einem Metalloxid, insbesondere Titandioxid und/oder Zinkoxid, einem Farbstoff, einem Leuchtstoff, und einem synthetischen Kunststoff, insbesondere Elastan und/oder Polyester;
wobei die intrinsischen Bestandteile und/oder die nicht-cellulosischen Bestandteile aus Alttextilien stammen.

15. Verwendung einer gemessenen und in einer Datenbank (152) hinterlegten Information hinsichtlich mindestens eines intrinsischen nichtcellulosischen Bestandteils, insbesondere einer Information hinsichtlich einer Kombination betreffend mehrere intrinsische nichtcellulosische Bestandteile, eines aus wiederverwerteten Alttextilien hergestellten Celluloseprodukts (150) oder eines Formkörpers (102), aus dem das Celluloseprodukt (150) hergestellt wurde, als Code zum Identifizieren einer Herkunft des Celluloseprodukts (150) oder des Formkörpers (102).
